# EUROPEAN PATENT APPLICATION

(11) **EP 1 897 886 A1**
(43) Date of publication of application: **12.03.2008**
(21) Application number: 06090164.2
(22) Date of filing: 08.09.2006
(51) Int. Cl.: C07K 7/00, C07K 14/00, A61K 38/04, A61K 38/16, A61K 47/48

(54) **Compounds as aptamer-dimers and their uses in diagnosis and therapy**

(71) Applicant: Bayer Schering Pharma Aktiengesellschaft, 13353 Berlin (DE)
(72) Inventor: Friebe, Matthias, 13509 Berlin (DE); Stephens, Andrew, Boulder, CO 80305 (US); Srinivasan, Ananth, 10629 Berlin (DE); Hecht, Maren, 10119 Berlin (DE)

(57) **Abstract**

The object of the present invention is solved by compositions of peptide linkers and their use for synthesis and annealing of aptamer oligonucleotides in form of dimers. The present invention in particularly provides said compositions for the use as a diagnostic and/or therapeutic agent.
In another preferred embodiment the present invention relates to a kit for preparing a radiopharmaceutical preparation, said kit comprising a vial comprising a quantity of the compound according to the invention.

A further preferred embodiment of the present invention relates to methods of producing said compounds, comprising synthesis of said compound in an automated peptide synthesizer.

In another preferred embodiment the present invention concerns the use of said compounds according to the present invention for the manufacture of a medicament, preferably for diagnosis or therapy of proliferative diseases.

In a further preferred embodiment the present invention concerns methods for treating or diagnosing patients administering or utilizing the compounds according to the present invention.

## Description

The present invention relates to the field of nucleic acids, and, more particularly, to compositions of oligonucleotides and their uses as diagnostic and/or therapeutic agent. In particular, the present invention relates to the synthesis of aptamer oligonucleotides in a dimeric format. The present invention further provides dimeric or multimeric aptamer probes with peptidic linkers. Those linkers can contain chelating units for metal binding or conjugation sites for other labelling approaches (e. g. dyes etc.). The invention further comprises chelator-aptamer conjugates, aptamer-dye conjugates and methods of targeted detection of analytes *in vitro* and *in vivo.* Such molecules are useful e. g. for molecular imaging applications, due to their rapid blood-clearance, tumor penetration, tumor imaging, and targeted delivery of radioisotopes. The molecules are also useful for tumor therapy and radiotherapy.

### BACKGROUND OF THE INVENTION

For the purposes of the present invention all references as cited herein are incorporated in their entireties by reference.

Aptamers are nucleic acid molecules having specific affinity for non-nucleic acid targets (e. g. proteins) or nucleic acid molecules through interactions other than classic Watson-Crick base pairing. Aptamers are described e.g., in U.S. Patent Nos. 5,475,096; 5,270,163; 5,589,332; and 5,741,679.

Aptamers are able to bind with high affinity and selectivity to many diverse types of target molecules, such as small molecules, peptides, proteins, viral particles and even whole cells (Jayasena S. D. (1999) Aptamers: an emerging class of molecules that rival antibodies in diagnostics. Clin. Chem., 45, 1628-1650; Sullenger B. A. and Gilboa, E. (2002) Emerging clinical applications of RNA. Nature, 418, 252-258; Rimmele M. (2003) Nucleic acid aptamers as tools and drugs: recent developments. Chembiochem, 4, 963-971; Lee J. F., Hesselberth,J. R., Meyers, L. A. and Ellington, A.D. (2004) Aptamer database. Nucleic Acids Res., 32, D95-D100) with dissociation constants down to picomolar values. This is due to their ability to form elaborate three-dimensional structures.
Consisting of nucleic acids, aptamers are selected from a large combinatorial library by a process of iterative selection and amplification. This method is referred to as Systematic Evolution of Ligands by Exponential Enrichment (SELEX) (Tuerk C. and Gold, L. (1990) Systematic evolution of ligands by exponential enrichment: RNA ligands to bacteriophage T4 DNA polymerase. Science, 249, 505-510; Ellington A. D. and Szostak, J. (1990) In vitro selection of RNA molecules that bind specific ligands. Nature, 346, 818-822; Daniels D. A., Chen, H., Hicke, B. J., Swiderek, K. M. and Gold, L. (2003) A tenascin-C aptamer identified by tumor cell Selex: systematic evolution of ligands by exponential enrichment. Proc. Natl Acad. Sci. USA, 100, 15416-15421). The molecular weight of aptamers (10-15 kDa in size, 30-40 nucleotides) is one order of magnitude lower than that of antibodies (150 kDa) (White R. R., Sullenger, B. A. and Rusconi, C. P. (2000) Developing aptamers into therapeutics. J. Clin. Invest., 106, 929-934), hence aptamers are expected to have rapid tissue and tumor penetration, as well as fast blood clearance. In contrast to antibodies, aptamers are non-immunogenic and can display favourable target-to-noise ratios at early time points. Thus, aptamers exhibit many characteristics desired for non-invasive *in vivo* tumor imaging and therapy (Hicke B. J. and Stephens, A. W. (2000) Escort aptamers: a delivery service for diagnosis and therapy. J. Clin. Invest., 106, 923-928; Charlton J., Sennello, J. and Smith, D. (1997) In vivo imaging of inflammation using an aptamer inhibitor of human neutrophil elastase. Chem. Biol., 4, 809-816; Drolet, D. W., Nelson, J., Tucker, C.E., Zack, P. M., Nixon, K., Bolin, R., Judkins, M. B., Farmer, J. A., Wolf, J. L., Gill, S. C. and Bendele, R. A. (2000) Pharmacokinetics and safety of an anti-vascular endothelial growth factor aptamer (NX1838) following injection into the vitreous humors of rhesus monkeys. Pharm. Res., 17, 1503-1510). In addition, aptamers, which are fully synthetic molecules, allow both rapid analoging for structure-activity relationship (SAR) studies and site-specific modification and conjugation for the attachment of unique chelating molecules, photoactive probes, radionuclides, drugs and pharmacokinetic modifying agents (Hilger S., Willis, M. C., Wolters, M. and Pieken, W. A. (1999) Tc-99m-labeling of modified RNA. Nucleosides Nucleotides, 18, 1479-1481; Hilger S., Willis, M. C., Wolters, M. and Pieken, W.A. (1998) Synthesis of Tc-99m labeled, modified RNA. Tetrahedron Lett., 39, 9403-9406; Kühnast, B., Dollé, F., Terrazzino, S., Rousseau, B., Loc'h, C., Vaufrey, F., Hinnen, F., Doignon, I., Pillon, F., David, C., Crouzel, C. and Tavitian, B. (2000) General method to label antisense oligonucleotides with radioactive halogens for pharmacological and imaging studies. Bioconjug. Chem., 11, 627- 636; Tavitian B. (2003) *In vivo* imaging with oligonucleotides for diagnosis and drug development. Gut, 52 (Suppl. 4), iv40-iv47).

Prerequisites for a successful *in vivo* application of aptamers as molecular targeting imaging agents are represented by high affinity and selectivity for their target as well as by adequate stability against *in vivo* degradation. A number of strategies have been employed to stabilize aptamers against 3' and 5' exonucleases as well as endonucleases, while maintaining target affinity. Chemical modifications at the 2' position of the ribose moiety, circularization of the aptamer, 3' capping and 'spiegelmer' technology have been described (White R. R., Sullenger, B. A. and Rusconi, C. P. (2000) Developing aptamers into therapeutics. J. Clin. Invest., 106, 929-934; Pieken W. A., Olsen, D. B., Benseler, F., Aurup, H. and Eckstein, F. (1991) Kinetic characterization of ribonuclease-resistant 2'-modified hammerhead ribozymes. Science, 253, 314-317; Jellinek D., Green, L. S., Bell, C., Lynott, C. K., Gill, N., Vargeese, C., Kirschenreuter, G., Mc Gee, D. P., Abesinghe, P., Pieken, W. A., Shapiro, R., Rifkin, D. B., Moscatelli, D. and Janjic, N. (1995) Potent 2'-amino-2'-deoxypyrimidine RNA inhibitors of basic fibroblast growth factor. Biochemistry, 34, 11363-11372; Klussmann S., Nolte, A., Bald, R., Erdmann, V. A. and Fuerste, J. P. (1996) Mirror-image RNA that binds d-adenosine. Nat. Biotechnol., 14, 1112-1115; Nolte A., Klussmann, S., Bald, R., Erdmann, V. A. and Furste, J. P. (1996) Mirror-design of 1-oligonucleotide ligands binding to L-arginine. Nat Biotechnol., 14, 1116-1119; Kim, S. J., Kim, M. Y., Lee, J. H., You, J. C. and Jeong, S. (2002) Selection and stabilization of the RNA aptamers against the human immunodeficiency virus type-1 nucleocapsid protein. Biochem. Biophys. Res. Commun., 291, 925-931).

One way to stabilize aptamers is to increase thermal stability of double-stranded areas located within non-binding regions. In this respect, locked nucleic acids (LNAs) hold great promise because of their substantially increased helical thermostability and excellent mismatch discrimination when hybridized with RNA or DNA (Petersen, M. and Wengel, J. (2003) LNA: a versatile tool for therapeutics and genomics. Trends Biotechnol., 21, 74-81; Koshkin A. A., Singh, S. K., Nielsen, P., Rajwanshi, V. K., Kumar, R., Meldgaard, M., Olsen, C. E. and Wengel, J. (1998) LNA (Locked Nucleic Acids) synthesis of the Adenine, Cytosine, Guanine, 5-Methylcytosine, Thymine and Uracil bicyclonucleoside monomers, oligomerisation, and unprecedented nucleic acid recognition. Tetrahedron, 54, 3607-3630; Koshkin A. A., Rajwanshi, V. K. and Wengel, J. (1998) Novel convenient synthesis of LNA [2.2.1] bicyclo nucleosides. Tetrahedron Lett., 39, 4381-4384; Braasch, D. A. and Corey, D. R. (2000) Locked nucleic acid (LNA): fine-tuning the recognition of DNA and RNA. Chem. Biol., 55, 1-7; Koshkin, A. A., Nielsen, P., Meldgaard, M., Rajwanshi, V. K., Singh, S. K. and Wengel, J. (1998) LNA (Locked Nucleic Acid): an RNA mimic forming exceedingly stable LNA:LNA duplexes. J. Am. Chem. Soc., 120, 13252-13253). A further advantage of LNA is its resistance to degradation by nucleases (Wahlestedt C., Salmi, P., Good, L., Kela, J., Johnsson, T., Hokfelt, T., Broberger, C., Porreca, F., Lai, J., Ren, K., Ossipov, M., Koshkin, A., Jakobsen, N., Skouv, J., Oerum, H., Jacobsen, M. H. and Wengel, J. (2000) Potent and nontoxic antisense oligonucleotides containing locked nucleic acids. Proc. Natl Acad. Sci. USA, 97, 5633-5638; Kumar R., Singh, S. K., Koshkin, A. A., Rajwanshi, V. K., Meldgaard, M. and Wengel, J. (1998) The first analogues of LNA (locked nucleic acids): phosphorothioate-LNA and 2'-thio-LNA. Bioorg. Med. Chem. Lett., 8, 2219-2222; Kurreck, J., Wysko, E., Gillen, C. and Erdmann, V. A. (2002) Design of antisense oligonucleotides stabilized by locked nucleic acids. Nucleic Acids Res., 30, 1911-1918). Based on these attractive features, the LNA modification has found successful applications in antisense oligonucleotides (Petersen M. and Wengel, J. (2003) LNA: a versatile tool for therapeutics and genomics. Trends Biotechnol., 21, 74-81; Frieden M., Christensen, S. M., Mikkelsen, N. D., Rosenbohm, C., Thrue, C. A., Westergaard, M., Hansen, H. F., Orum, H. and Koch, T. (2003) Expanding the design horizon of antisense oligonucleotides with alpha-L-LNA. Nucleic Acids Res., 31, 6365-6372; Jepsen J. S. and Wengel, J. (2004) LNA-antisense rivals siRNA for gene silencing. Curr. Opin. Drug Discov. Devel., 7, 188-194; Hansen J. B., Westergaard, M., Thrue, C. A., Giwercman, B. and Oerum, H. (2003) Antisense knockdown of PKC-alpha using LNA-oligos. Nucleosides Nucleotides 22, 1607-1609; Grunweller A., Wyszko, E., Bieber, B., Jahnel, R., Erdmann, V. A. and Kurreck, J. (2003) Comparison of different antisense strategies in mammalian cells using locked nucleic acids, 2'-O-methyl RNA, phosphorothioates and small interfering RNA. Nucleic Acids Res., 31, 3185-3193), DNAzymes (Vester B., Lundberg, L. B., Sorensen, M. D., Babu, R., Douthwaite, S. and Wengel, J. (2002) LNAzymes: incorporation of LNA-type monomers into DNAzymes markedly increases RNA cleavage. J. Am. Chem. Soc., 124, 13682-13683; Schubert S., Gul, D. C., Grunert, H. P., Zeichhardt, ., Erdmann, V. A. and Kurreck, J. (2003) RNA leaving `10-23' DNAzymes with enhanced stability and activity. Nucleic Acids Res., 31, 5982-5992) and decoy oligonucleotides (Crinelli, R., Bianchi, M., Gentillini, L. and Magnani, M. (2002) Design and characterization of decoy oligonucleotides containing locked nucleic acids. Nucleic Acids Res., 30, 2435-2443). Recent studies show that LNA modification within aptamers is very promising with regard to nucleolytic stability (Darfeuille F., Hansen, J. B., Orum,H., Di Primo, C. and Toulme, J. J. (2004) LNA/DNA chimeric oligomers mimic RNA aptamers targeted to the TAR RNA element of HIV-1. Nucleic Acids Res., 32, 3101-3107). In our attempts to generate new powerful probes for *in vivo* imaging applications, we applied the LNA modification to improve the Tenascin-C aptamer TTA1 (Hicke, B. J., Marion, C., Chang, Y.-F., Gould, T., Lynott, C. K., Parma, D., Schmidt, P. G. and Warren, S. (2001) Tenascin-C aptamers are generated using tumor cells and purified protein. J. Biol. Chem., 276, 48644-48654) with regard to *in vivo* stability, targeting function and bio distribution. TTA1 is a 39mer oligonucleotide (molecular weight of 13.4 kDa) that structurally recognizes human Tenascin-C (TN-C) and binds to it with high affinity (*K*_{d} of 5 × 10⁻⁹ M) (Hicke B. J., Marion, C., Chang, Y.-F., Gould, T., Lynott, C. K., Parma, D., Schmidt, P. G. and Warren, S. (2001) Tenascin-C aptamers are generated using tumor cells and purified protein. J. Biol. Chem., 276, 48644-48654). TN-C is a hexameric protein found in the extracellular matrix that plays an important role in tumorigenesis, embryogenesis and wound healing (Erickson, H. P. and Bourdon, M. A. (1989) Tenascin: an extracellular matrix protein prominent in specialized embryonic tissues and tumors. Annu. Rev. Cell Biol., 5, 71-92). Based on this feature, TTA1 labeled with technetium-99m (Tc-99m) is a promising candidate for imaging of tumors expressing TN-C. The TTA aptamer is currently in clinical trials. Adequate stability of TTA1 against nucleolytic degradation has been achieved by replacement of all pyrimidine ribonucleotides by 2'-deoxy-2'-fluoro nucleotides and 14 of the 19 purine ribonucleotides by 2'-deoxy-2'-OMe nucleotides. Additionally, the 3' end is blocked with a 3'-3'-thymidine cap. Further structural characteristics include a (CH₂CH₂O)₆ spacer and a 5' hexyl-aminolinker. Via the latter, an N₂S peptidyl radiometal chelate (Hilger, S., Willis, M. C., Wolters, M. and Pieken, W. A. (1999) Tc-99m-labeling of modified RNA. Nucleosides Nucleotides, 18, 1479-1481; Hilger, S., Willis, M. C., Wolters, M. and Pieken, W. A. (1998) Synthesis of Tc-99m labeled, modified RNA. Tetrahedron Lett., 39, 9403-9406) can be attached to TTA1. Limited SAR studies have been performed so far for TTA1.

Aptamers are often characterized by a rapid renal clearance, with half-lifes of < 10 minutes, leading to high target to non-target ratios (Healy, J. M., Lewis, S. D., Kurz, M., Boomer, R. M., Thompson, K. M., Wilson, C., McCauley, T. G. (2004) Pharmacokinetics and Biodistribution of Novel Aptamer Compositions, Pharmaceutical Research, 21, 2234-2246). On the other hand, this rapid elimination of the compound may need some fine-tuning to assure a sufficient *in vivo* residence time for an optimal accumulation in the target organ or the respective binding site. The urinary filtration can be reduced by e.g. defined increase in molecular weight.

A tailor-made molecular weight would be an easy modification handle to control the compounds residence time *in vivo.* The most common way to alter the molecular weight of oligonucleotides is achieved by the introduction of polyethylene glycol (PEG) groups into the molecule (Watson, S. R., Chang, Y. F., O'Connell, D., Weigand, L., Rinquist, S., Parma, D. H. (2000) Anti-L-selectin aptamers: binding characteristics, pharmacokinetic parameters and activity against an intravascular target in vivo. Antisense Nucleic Acid Drug Dev, 10, 63-75). The introduction of a hydrophilic chain in the back-bone of the aptamer may also direct the compound more towards a renal elimination in addition to an defined increase in molecular weight.

Dimerization is another option to increase the weight of an aptamer, besides the introduction of PEGylated chains. Dimeric aptamers are characterized by an increased molecular weight extending the blood retention and the tissue penetration time. Furthermore, a dimer can potentially bind bivalently and thus more avidly to the target protein. This more avid binding could improve the tumor targeting properties by means of a slower dissociation from the target molecule. Considering that every tenascin-C matrix protein has six possible binding sites, a dimeric aptamer could bind bivalently to one target molecule, yielding lower off-rates compared to the monomeric form. David Parma and Steven Ringquist showed that a bivalent aptamer, assayed against L-selectin-expressing lymphocytes, exhibited an enhanced affinity and a slower off-rate from the target than the univalent aptamer (Ringquist, S., and Parma, D. (1998) Anti-L-Selectin Oligonucleotide Ligands Recognize CD62L-Positive Leukocytes: Binding Affinity and Specificity of Univalent and Bivalent Ligands, Cytometry, 33, 394-405).

A chelate is a water-soluble complex between a metal ion and a complexing agent. It usually does not dissociate easily in solution, but forms an inert complex. In labile complexes, however, the metal ion can be readily exchanged. Metal complexes of transition elements are well known, chelation occurs within a much wider range of elements. Chelating agents yielding soluble metal complexes are also called sequestering agents (Chaberek, S., and Martell, A. E. Organic Sequestering Agents, Wiley, New York 1959). A chelating agent has at least two functional groups which donate a pair of electrons to the metal, such as = O, -NH₂ or -COO⁻. Furthermore, these groups must be located so as to allow ring formation with the metal. Chelating agents are widely found in living systems and are of importance in cellular metabolism.

The metal chelating residue serve the purpose of binding to metals in particular to metal ions and to recruit such metal ions to the tissues. A large variety of such metal chelating moieties are known in the art and are described in, for example, US 5,654,272, US 5,681,541, US 5,788,960, US 5,811,394, US 5,720,934, US 5,776,428, US 5,780,007, US 5,922,303, US 6,093,383, US 6,086,849, US 5,965,107, US 5,300,278, US 5,350,837, US 5,589,576, US 5,679,778, US 5,789,659, and US 6,358,491.

It is therefore an object of the present invention, to provide improved aptamer molecules that have particular advantages when used in radiodiagnosis and/or therapy.

### SUMMARY OF THE INVENTION

The object of the present invention is solved by compositions of peptide linkers and their use for synthesis and annealing of aptamer oligonucleotides in form of dimers. The present invention in particularly provides said compositions for the use as a diagnostic and/or therapeutic agent.

In one preferred aspect thereof, the present invention provides a compound of general formula (I)

Mal-L₁-(A)ₙ-C-(A)ₘ-L₂-Mal (formula I)

wherein Mal is maleimide; L₁ and L₂ each are absent or a linker structure selected from branched or unbranched, substituted or unsubstituted (C₁-C₁₂)Alkyl, (C₁-C₁₂)Alkoxy, e.g. ethylene glycolₒ wherein o is and integer from 1 to 12, wherein L₁ and L₂ can be the same or different; A is an amino acid or an derivative thereof; n, m independently are selected from an integer between 0 and 20; C is absent or a metal ion chelating group; or is a linking group for conjugating of a halogen bearing synthon, wherein A and/or C independently can comprise one or more, identical or different protecting groups, and pharmaceutically acceptable salts thereof.

Another preferred embodiment of the present invention provides a compound of general formula (II)

T₁-Mal-L₁-(A)ₙ-C-(A)ₘ-L₂-Mal-T₂ (formula II)

Wherein T₁ and T₂ each are a targeting group, wherein T₁ and T₂ independently can be identical or different; Mal is maleimide; L₁ and L₂ each are absent or a linker structure selected from branched or unbranched, substituted or unsubstituted (C₁-C₁₂)Alkyl, (C₁-C₁₂)Alkoxy, e.g. ethylene glycolₒ wherein o is and integer from 1 to 12, wherein L₁ and L₂ can be the same or different; A is an amino acid or an derivative thereof, n, m independently are selected from an integer between 0 and 20; C is absent or a metal ion chelating group; or is a linking group for conjugating of a halogen bearing synthon; wherein A and/or C independently can comprise one or more, identical or different protecting groups, and pharmaceutically acceptable salts thereof.

In another preferred embodiment the present invention relates to a kit for preparing a radiopharmaceutical preparation, said kit comprising a vial comprising a quantity of the compound according to the invention.

A further preferred embodiment of the present invention relates to methods of producing said compounds, comprising synthesis of said compound in an automated peptide synthesizer.

In another preferred embodiment the present invention concerns the use of said compounds according to the present invention for the manufacture of a medicament, preferably for diagnosis or therapy of proliferative diseases.

In a further preferred embodiment the present invention concerns methods for treating or diagnosing patients administering or utilizing the compounds according to the present invention.

### DETAILED DESCRIPTION OF THE PRESENT INVENTION

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W., Nagel, B. and Kölbl, H. eds. (1996),Wiley-VCH, Weinheim.

Throughout this specification and the claims which follows, unless the context requires otherwise, the word "comprise", and variations such as "comprises" and "comprising", will be understood to imply the inclusion of a stated integer or step or group of integers or steps, but not the exclusion of any other integer or step or group of integer or step.

As outlined above there is a need in the prior art to provide compounds with increased tumor accumulation and retention and an increased half-life *in vivo* for the use as a diagnostic and/or therapeutic agent. The present invention is based on compositions of peptide linkers and their use for synthesis and annealing of aptamer oligonucleotides in form of dimers or higher multimers. Such molecules are useful for molecular imaging applications including rapid tumor penetration, blood clearance, tumor radioimaging, targeted delivery of radioisotopes. The molecules are also useful for tumor and general therapy.

In a first aspect the present invention provides a compound of general formula (I)

Mal-L₁-(A)ₙ-C-(A)ₘ-L₂-Mal (formula I)

Wherein Mal is maleimide. It may be referenced herein in abbreviated form as N.

L₁ and L₂ each are absent or a linker structure selected from branched or unbranched, substituted or unsubstituted (C₁-C₁₂)Alkyl, (C₁-C₁₂)Alkoxy, e.g. ethylene glycolₒ wherein o is and integer from 1 to 12, wherein L₁ and L₂ can be the same or different. Within the scope of this invention, branched or unbranched, substituted or unsubstituted "alkyl" is, for example, methyl, ethyl, propyl, isopropyl, butyl, isobutyl, sec-butyl, pentyl, isopentyl or hexyl, heptyl, octyl, nonyl, decyl, undecyl, or dodecyl.

Within the scope of this invention, "alkoxy" is, for example, methyloxy, ethyloxy, propyloxy, isopropyloxy, butyloxy, isobutyloxy, sec-butyloxy, pentyloxy, isopentyloxy, hexyloxy, heptyloxy, octyloxy, nonyloxy, decyloxy, undecyloxy or dodecyloxy.

The term "linker" refers to a chemical constituent which is covalently attached at one point to the maleimide. The linker itself can consist of many different components, each having a characteristic property offering a unique advantage. One skilled in the art of organic synthesis could design and synthesize a great variety of linkers having the required functionality. A wide array of appropriate linkers can be designed and synthesized through the application of routine experimentation (Sandler and Karo, Polymer Synthesis Vol. 1, Academic Press, Inc. (1992); Sandler and Karo, Polymer Synthesis Vol. 2, Academic Press, Inc. (1994)).

A is an amino acid or sequence thereof or an amino acid derivative.

By the term "amino acid" is meant an L- or D-amino acid, amino acid analogue or amino acid mimetic which may be naturally occurring or of purely synthetic origin, and may be optically pure, i.e. a single enantiomer and hence chiral, or a mixture of enantiomers. Preferably the amino acids of the present invention are optically pure. The term "amino acid" refers to very small biomolecules with an average molecular weight of about 135 daltons. These organic acids exist naturally in a zwitterion state where the carboxylic acid moiety is ionized and the basic amino group is protonated. The entire class of amino acids has a common backbone of an organic carboxylic acid group and an amino group attached to a saturated carbon atom. The simplest member of this group is glycine, where the saturated carbon atom is unsubstituted, rendering it optically inactive. The rest of the 20 most common amino acids are optically active existing as both D and L stereoisomers. Naturally occurring amino acids that are incorporated into proteins are, for the most part, the levorotary (L) isomer. Substituents on the alpha (or saturated) carbon atom vary from lower alkyl groups to aromatic amines and alcohols. There are also acidic and basic side chains as well as thiol chains that can be oxidized to dithiol linkages between two similar amino acids.

If the amino acids of the compounds of the formula I can occur in a plurality of enantiomeric forms, all these forms and also mixtures thereof (for example the DL forms) are included.

In addition, also structural elements like *N*-terminal modified or carboxy-terminal modified derivatives are part of this invention. Favored are amino-terminal methyl-, ethyl-, propyl-, butyl-, tert-butyl-, neopentyl-, phenyl- or benzyl-groups, amino-terminal groups like BOC, Mtr, CBZ, Fmoc, and, particularly, acetyl, benzoyl or (indol-3-yl)carbonic acid groups, furthermore, carboxy-terminal methyl-, ethyl-, propyl-, butyl-, tert-butyl-, neopentyl- or benzylester, methyl-, ethyl-, propyl-, butyl-, tert-butyl-, neopentyl- or benzylamides and, particularly, carboxamides.

C is absent or a metal ion chelating group; or is a linking group for conjugating of a halogen bearing synthon.

The term "metal ion chelator" refers to organic chemicals that form two or more coordination bonds with a central metal ion. Heterocyclic rings are formed with the central metal atom as part of the ring. Some biological systems form metal chelates, e.g., the iron-binding porphyrine group of hemoglobin and the magnesium-binding chlorophyll of plants. (From Hawley's Condensed Chemical Dictionary, 12th ed).

The term "synthon" shall mean any pre-formulated chemical structure bearing a halogen atom suitable of being connected to the compound of formula (I) via C, if C is selected to be a linker capable of being connected to said synthon. Examples of such synthons are, but are not limited to, N-Succinimidyl-4-[¹⁸F]fluorobenzoate (SFB), N-Succinimidyl-3-(4-hydroxy-3-[¹²⁵I]iodophenyl)propionate ([¹²⁵I] Bolton & Hunter reagent, Ref.: J Russell, JA O'Donoghue, R Finn, J Koziorowski, S Ruan, JL Humm, CC Ling, (2002) Iodination of Annexin V for Imaging Apoptosis, The Journal of Nuclear Medicine, 43 671-677), N-(4-[¹⁸F]fluorobenzyl)-2-bromoacetamide (Ref.: F Dollé, F Hinnen, F Vaufrey, B Tavitian, C Crouzel, (1997) A general method for labeling Oligodeoxynucleotides with 18F for in vivo PET imaging, Journal of Labeled Compounds and Radiopharmaceuticals, 39 319-330), N-{4-[(4-[¹⁸F]fluorobenzylidene)aminooxy]butyl}-maleimide (Ref.: T Toyokuni, JC Walsh, A Dominguez, ME Phelps, JR Barrio, SS Gambhir, N Satyamurthy, (2003) Synthesis of a new Heterobifunctional Linker, N-[4-(Aminooxy)butyl]maleimide, for Facile Access to a Thiol-Reactive 18F-Labeling Agent, Bioconjugate Chemistry, 14 1253-1259), 1-[3-(2-[¹⁸F]fluoropyridin-3-yloxy)-propyl]-pyrrole-2,5-dione, 4-[¹⁸F]fluorobenzaldehyde, and Bromo[¹⁸F]fluoroethane.

n, m each are independently an integer between 0 and 20; wherein any A and/or C can have independently of each other, one or more, same or different, protecting groups attached.

The term "protecting groups" is known in general terms and relates to groups which are suitable for protecting (blocking) an amino group against chemical reactions, but are easy to remove, i.e. a group which inhibits or suppresses undesirable chemical reactions, but which is designed to be sufficiently reactive that it may be cleaved from the functional group in question under mild enough conditions that do not modify the rest of the molecule. Typical for such groups are, in particular, unsubstituted or substituted acyl, aryl, aralkoxymethyl or aralkyl groups. Furthermore, the amino acids may, for example, as a constituent of compounds of the formula I, be provided with corresponding protecting groups known per se. Favored are derivatives of Asp and Glu, particularly methyl-, ethyl-, propyl-, butyl-, *tert*-butyl-, neopentyl- or benzylester of the side chain or derivatives of Tyr, particularly methyl-, ethyl-, propyl-, butyl-, *tert*-butyl-, neopentyl- or benzylethers of the side chain. Protecting groups are well known to those skilled in the art and are suitably chosen from: Boc (where Boc is tert-butyloxycarbonyl), Fmoc (where Fmoc is fluorenylmethoxycarbonyl), trifluoroacetyl, allyloxycarbonyl, Dde [i.e. 1-(4,4-dimethyl-2,6-dioxocyc- lohexylidene)ethyl] or Npys (i.e. 3-nitro-2-pyridine sulfenyl); methyl ester, tert-butyl ester, benzyl ester; acetyl, benzoyl, trityl (Trt) or tetrabutyldimethylsilyl. Other suitable protecting groups are: Trityl and 4-methoxybenzyl. The use of further protecting groups are described in 'Protective Groups in Organic Synthesis', Greene, Theorodora W., and Wuts, Peter G. M. (John Wiley & Sons, 1991).

The compounds of formula I may be partially or completely synthesized, for example using solution or solid state synthesis techniques known in the art (Gysin, B. F., and Merrifield, R. B. (1972) J. Am. Chem. Soc., 94, 3102; or Merrifield, R. B. (1985) Angew. Chemie Int. Ed., 24(10), 799-810) applying appropriate amino or carboxy building blocks. A sequential synthesis is contemplated. Other organic synthetic methods may be employed in the synthesis of the compounds according to formula I, such as the methods described in Houben-Weyl, Methods of Organic Chemistry, Georg-Thieme-Verlag, Stuttgart.

In a preferred embodiment of the compound of general formula (I) A is according to the present invention selected from natural and/or unnatural amino acids.

Within the context of the present invention A can mean any naturally occurring amino acids. These amino acids may be referenced herein in abbreviated form, using standard one-letter or three-letter codes (which can be found, for example, in G. Zubay, Biochemistry (2d. ed.), 1988, Mac Millan Publishing: New York, p.33). The term "non-natural amino acids" comprise substituted derivatives of natural occurring amino acids.

In accordance with the present invention, a "substituted derivative" of an amino acid includes such substitutions as amino, hydroxyl, N-alkyl wherein alkyl represents C₁ to C₄ alkyl, N-aryl, N-acyl, O-alkyl wherein alkyl represents C₁ to C₄ alkyl, O-aryl, O-acyl, S-alkyl wherein alkyl represents C₁ to C₄ alkyl, S-aryl. When applied to amino acids that contain a side chain aromatic ring, the term also encompasses o-, m-, and p-substitutions including but not limited to o-amino, m-amino, p-amino, amino, o-hydroxyl, m-hydroxyl, p-hydroxyl, and the like.

Particularly preferred natural or unnatural amino acids which can constitute (A)ₙ or (A)ₘ are alanine, asparagine, aspartic acid, glutamine, glutamic acid, phenylalanine, glycine, histidine, isoleucine, lysine, leucine, proline, arginine, serine, threonine, tryptophane, valine, tyrosine, tert-butyl glycine, N-methyl phenylalanine (NMe)Phe, Hcy, Hhc, Pen, Aib, Nal, Aca, Ain, Hly, Achxa, Amf, Aec, Apc, Aes, Aps, Abu, Nva, FD, WD, YD, Cpa, Thp, D-Nal, Dpg, Nle, (1)Nal, (2)Nal, (N-CH₃)Cys, (N-CH₃)Hcy, (N-CH₃)Tyr, (N-CH₃)Tty, (N-CH₃)Tyr(CH₂CH₂SH), Tpi, Thr(OH), Ser(ol), Asp(ol), Glu(ol), Gln(ol), Asn(ol), (4)PaL, Phe(4-F), Phe(4-NH₂), ε-Leusine, δ-Orn, γ-Dab and β-Dab. As used herein, the following amino acids and amino acid analogues are intended to be represented by the following abbreviations: Hcy is homocysteine; Hhc is homohomocysteine (3-mercaptopropylglycine); Pen is penicillamine; Aib is aminoisobutyric acid; Nal is 2-naphthylalanine; Aca is 6-aminocaproic acid; Ain is 2-aminoindan-2-carboxylic acid; Hly is homolysine; Achxa is 4-amino-cyclohexylalanine; Amf is 4-aminomethyl-phenylalanine; Aec is S-(2-aminoethyl)cysteine; Apc is S-(3-aminopropyl) cysteine; Aes is O-(2-aminoethyl)serine; Aps is O-(3-aminopropyl)serine; Abu is 2-aminobutyric acid; Nva is norvaline; F*_{D}* is D-phenylalanine; W*_{D}* is D-tryptophan; Y*_{D}* is D-tyrosine; Cpa is L-(4-chlorophenyl) alanine; Thp is 4-amino-tetrahydrothiopyran-4-carboxylic acid; D-Nal is D-2-naphthylalanine; Dpg is dipropylglycine; Nle is norleucine; (N-CH₃)Cys is N-methyl-cysteine; (N-CH₃)Hcy is N-methyl-homocysteine; (N-CH₃)Tyr is N-methyl-tyrosine; (N-CH₃)Tty is N-methyl-thiotyrosine (i.e., N-methyl-4-mercaptophenylalanine); (N-CH₃)Tyr(CH₂ CH₂ SH) is N-methyl-0-2-mercaptoethyl tyrosine; Thr(OH) is threoninol residue (wherein the carboxyl group of the amino acid is reduced to a primary alcohol, incorporated into the peptide using the procedure of Neugebauer et al. (1990, Peptides: Proceedings of the 11th American Peptide Symposium, pp. 1020-21); Ser(ol) is. serinol; Asp(ol) is aspartinol; Glu(ol) is glutarinol; Gln(ol) is glutaminol; Asn(ol) is asparaginol; Phe(4-F) is 4-fluoro-phenylalanine; Phe(4-NH₂) is 4-amino-phenyalanine; ε-Lys represents a covalent linkage via the ε-amino group on the side chain of a lysine residue; δ-Orn represents an ornithine residue in which the δ-amino group is covalently linked to the carboxyl group of the adjacent amino acid to form a peptide bond; γ-Dab represents a 2,4-diaminobutyric acid residue in which the γ-amino group is covalently linked to the carboxyl group of the adjacent amino acid to form a peptide bond; β-Dap represents a 2,3-diaminopropionic acid residue in which the β-amino group is covalently linked to the carboxyl group of the adjacent amino acid to form a peptide bond.

In a more preferred embodiment of the present invention at least one A of the compound of general formula (I) is according to the present invention a D-amino acid.

The term "D-amino acid" refers to stereoisomers of naturally occurring L-amino acids. D-amino acids are never found in proteins, although they exist in nature. D-amino acids are often found in polypetide antibiotics. Natural proteins almost exclusively uses laevorotatory or left-handed amino acids, rather than D-enantiomers. However, with improved analytical methods, D-amino acids have been detected in a variety of peptides of multicellular organisms. The resulting molecules are nonetheless not inert, but rather may induce a unique immune response, which is hardly cross- reactive with the L-enantiomer. Moreover, cross-reaction between the L- and the D-sequences is limited also at the T cell level, probably due to different sterical conformations of the MHC-antigen-T cell receptor complexes formed. Polypeptides built exclusively of D-amino acids lead to antibody formation only at a relatively low concentration, otherwise they provoke immunological paralysis.

It is particularly preferred that the integer n and m of the compound according to the present invention is the same and is preferably selected from 0, 1, 2, 3, 4, and 5.

In another preferred embodiment according to the present invention the C of the said compound is an N₃S chelator.

The term "N₃S chelator" refers to a triamide mercaptide which is a thiol-containing bifunctional chelator. As one example of a N₃S chelator, MAG(3) has been used successfully for labelling peptides, proteins, DNAs and other carriers with 99mTc, i.e. the class of triamide mercaptide (N₃S) was developed as Tc-99m-chelating agents (Fritzberg et al.,"Synthesis and biological evaluation of Tc-99m-MAG3 as a hippuran replacement" J. Nucl. Med. 27, 111-116 (1986)). To date, the 99mTc- mercaptoacetyltriglycine ([99mTcO-MAG3]-) is considered to be one of the most successful agents for functional renal imaging. In principle, N₃S chelators may be used to incorporate any natural or unnatural amino acids. It may be more convenient for some applications to conjugate the chelating agent N₃S to the polypeptide or carbohydrate in the absence of the metal ion. For example, since some of the radionuclides contemplated by the present invention (e.g., 99mTc) have relatively short half-lives, it may be desirable to prepare the chelating-agent-conjugated polypeptide or carbohydrate moiety and then, shortly prior to administration, react the chelating compound-conjugated polypeptide or carbohydrate moiety with the radionuclide of interest. For example, the carboxylic acid group would be linked to the polypeptide to form an amide linkage, followed by the addition of the metal, in a weakly complexed form. Alternatively, for chelating compounds having a free primary amine group, the N₃S chelating compounds could be linked to a carbohydrate moiety, as discussed above, by linking the primary amine to the carbohydrate moiety following periodate treatment of the carbohydrate moiety, under mild reducing conditions to form a secondary or tertiary amine linkage; this procedure would then be followed by the appropriate reaction with the radionuclide to form the desired radiolabeled carbohydrate moiety.

Initially, the metal ion might non-specifically as well as specifically bind to the polypeptide or carbohydrate. However, the N₃S ligand center should form chelates of higher stability than the non-specific sites, and the metal ions under equilibrium conditions would migrate to the N₃S chelating group. Those metal ions not bound to the N₃S chelating group could be washed away under mild conditions with a chelating agent, e.g., EDTA, or a non-ionic detergent. Conveniently, the metal ion could be added as a weakly chelated ion or in the presence of a weakly chelating group, such as a uronate, e.g., gluconate, or tartrate

In a more preferred embodiment according to the present invention the C of the said compound is selected from metal ion chelators of the group Dpr-Met-Cys and Dpr-Gly-Cys.

Peptidic linkers that contain the chelating unit Dpr-Met-Cys or Dpr-Gly-Cys can be synthesized in an automated peptide synthesizer. When the chelating site in the peptide sequence is formed by Dpr-Gly-Cys and Dpr-Met-Cys, peptides that contain the amino acid methionine instead of glycine offer an additional side chain in the chelating sequence that can add additional stability to the metal complex. This is due to the lone electron pair of the sulfur atom in ethyl sulfanyl methane that can serve as an additional donor. This chelating sequence is abutted on two spacer sequences which can be varied in length and in hydrophilicity in order to study the influence of these factors on conjugation success and plasma stability properties *in vitro.*

In a more preferred embodiment according to the present invention the L₁ of the said compound is the same as L₂ and is -CH₂-CH₂-CO-.

In a most preferred embodiment according to the present invention the compound is selected from the group of
Mal-CH₂-CH₂-CO-βAla-Ser-Dpr-Met-Cys(tButylthiol)-Ser-Lys-CO-CH₂-CH₂-Mal
Mal-CH₂-CH₂-CO-βAla-Ser-Dpr-Gly-Cys(tButylthiol)-Ser-Lys-CO-CH₂-CH₂-Mal
Mal-CH₂-CH₂-CO-βAla-Ser-Ser-Dpr-Met-Cys(tButylthiol)-Ser-Ser-Lys-CO-CH₂-CH₂-Mal
Mal-CH₂-CH₂-CO-βAla-Ser-Ser-Dpr-Gly-Cys(tButylthiol)-Ser-Ser-Lys-CO-CH₂-CH₂-Mal
Mal-CH₂-CH₂-CO-βAla-Ser-Ser-Ser-Dpr-Met-Cys(tButylthiol)-Ser-Ser-Ser-Lys-CO-CH₂-CH₂-Mal
Mal-CH₂-CH₂-CO-βAla-D-Ser-D-Ser-D-Ser-Dpr-Met-Cys(tButylthiol)-D-Ser-D-Ser-D-Ser-Lys-CO-CH₂-CH₂-Mal
Mal-CH₂-CH₂-CO-βAla-D-Ser-D-Ser-D-Ser-Dpr-Gly-Cys(tButylthiol)-D-Ser-D-Ser-D-Ser-Lys-CO-CH₂-CH₂-Mal
Mal-CH₂-CH₂-CO-βAta-Ser-Dpr-Met-Cys-Ser-Lys-CO-CH₂-CH₂-Mal
Mal-CH₂-CH₂-CO-βAla-Ser-Dpr-Gly-Cys-Ser-Lys-CO-CH₂-CH₂-Mal
Mal-CH₂-CH₂-CO-βAla-Ser-Ser-Dpr-Met-Cys-Ser-Ser-Lys-CO-CH₂-CH₂-Mal
Mal-CH₂-CH₂-CO-βAla-Ser-Ser-Dpr-Gly-Cys-Ser-Ser-Lys-CO-CH₂-CH₂-Mal
Mal-CH₂-CH₂-CO-βAla-Ser-Ser-Ser-Dpr-Met-Cys-Ser-Ser-Ser-Lys-CO-CH₂-CH₂-Mal
Mal-CH₂-CH₂-CO-βAla-D-Ser-D-Ser-D-Ser-Dpr-Met-Cys-D-Ser-D-Ser-D-Ser-Lys-CO-CH₂-CH₂-Mal
Mal-CH₂-CH₂-CO-βAla-D-Ser-D-Ser-D-Ser-Dpr-Gly-Cys-D-Ser-D-Ser-D-Ser-Lys-CO-CH₂-CH₂-Mal, and pharmaceutically acceptable salts thereof.

A particularly preferred compound according to the invention is

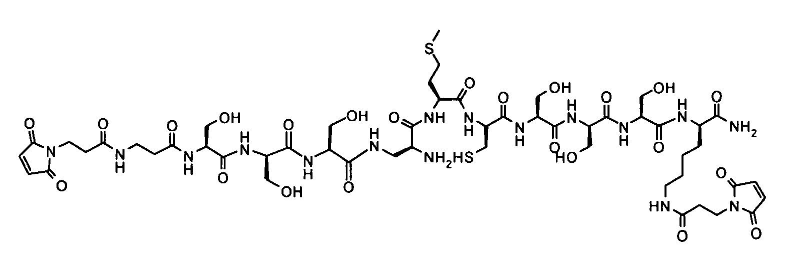

In a second aspect thereof, the present invention provides a compound of the general formula (II)

T₁-Mal-L₁-(A)ₙ-C-(A)ₘ-L₂-Mal-T₂ (formula II)

Wherein T₁ and T₂ each are a targeting group, wherein T₁ and T₂ independently can be identical or different; Mal is maleimide; L₁ and L₂ each are absent or a linker structure selected from branched or unbranched, substituted or unsubstituted (C₁-C₁₂)Alkyl, (C₁-C₁₂)Alkoxy, e.g. ethylene glycolₒ wherein o is and integer from 1 to 12, wherein L₁ and L₂ can be the same or different; A is an amino acid or an derivative thereof, n, m independently are selected from an integer between 0 and 20; C is absent or a metal ion chelating group; or is a linking group for conjugating of a halogen bearing synthon; wherein A and/or C independently can comprise one or more, identical or different protecting groups, and pharmaceutically acceptable salts thereof.

The term "targeting moiety" refers to a compound which functionally interacts with a binding site on a target causing a physical association between the targeting moiety and/or a conjugate attached to the targeting moiety and the surface of the target. Thus, the targeting moiety provides specificity for or binding affinity for one or more type of targets.

In a preferred embodiment of the compound of general formula (II) according to the present invention T1 and T2 are the same.

In another preferred embodiment of the compound of general formula (II) according to the present invention at least one of T1 or T2 is selected from the group of an aptamer oligonucleotide, a peptide, an antibody or a fragment thereof.

The term "aptamer oligonucleotide" refers to a nucleic acid molecule that is capable of binding to a particular molecule of interest with high affinity and specificity (Tuerk and Gold, (1990) Science 249, 505; Ellington and Szostak, (1990) Nature 346, 818). The binding of a ligand to an aptamer, which is typically RNA, changes the conformation of the aptamer. The conformation change inhibits translation of an mRNA in which the aptamer is located, for example, or otherwise interferes with the normal activity of the nucleic acid. Aptamers may also be composed of DNA or may comprise non-natural nucleotides and nucleotide analogs. An aptamer will most typically have been obtained by in vitro selection for binding of a target molecule. However, in vivo selection of an aptamer is also possible.

Aptamers have specific binding regions which are capable of forming complexes with an intended target molecule in an environment wherein other substances in the same environment are not complexed to the nucleic acid. The specificity of the binding is defined in terms of the comparative dissociation constants (Kd) of the aptamer for its ligand as compared to the dissociation constant of the aptamer for other materials in the enviromnent or unrelated molecules in general. Typically, the Kd for the aptamer with respect to its ligand will be at least about 10-fold less than the Kd for the aptamer with unrelated material or accompanying material in the environment. Even more preferably, the Kd will be at least about 50-fold less, more preferably at least about 100-fold less, and most preferably at least about 200-fold less. An aptamer will typically be between about 10 and about 300 nucleotides in length. More commonly, an aptamer will be between about 30 and about 100 nucleotides in length.

The terms "peptide" and "protein" can be used interchangeably, and refer to compounds comprised of at least two amino acid residues covalently linked by peptide bonds or modified peptide bonds (e.g., peptide isosteres). No limitation is placed on the maximum number of amino acids which may comprise a protein or peptide. The amino acids comprising the peptides or proteins described herein and in the appended claims are understood to be either D or L amino acids with L amino acids being preferred. The amino acid comprising the peptides or proteins described herein may also be modified either by natural processes, such as posttranslational processing, or by chemical modification techniques which are well known in the art. Modifications can occur anywhere in a peptide, including the peptide backbone, the amino acid side-chains and the amino or carboxyl termini. It is understood that the same type of modification may be present in the same or varying degrees at several sites in a given peptide. Also, a given peptide may contain many types of modifications. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, "Proteins-Structure and Molecular Properties", 2nd Ed., T. E. Creighton, W.H. Freeman and Company, New York, 1993 and Wold, F., Posttranslational Protein Modifications: Perspectives and Prospects, 1-12 in "Posttranslational Covalent Modification of Proteins", B. C. Johnson, Ed., Academic Press, New York, 1983; Seifter et al., (1990) Analysis for protein modifications and non-protein cofactors, Meth. Enzymol. 182, 626-646 and Rattan et al., (1992) Protein Synthesis: Posttranslational Modifications and Aging, Ann N.Y. Acad Sci 663, 48-62, the entire disclosures of which are herein incorporated by reference.

The term "antibody and a fragment thereof refers to any polypeptide or protein comprising at least one antigen binding domain which is, or is homologous to, an antibody antigen binding domain. The term "antigen binding domain" describes the part of an antibody or fragment which comprises the area which specifically binds to and is complementary to part or all of the antigen. Where an antigen is large, an antibody may bind only to a particular part of the antigen, which part is termed the epitope. An antigen binding domain may comprise one or more antibody variable domains. Preferably, an antigen binding domain comprises an antibody light chain variable region (VL) and an antibody heavy chain variable region (VH). Within the variable regions VH and VL, the parts that determine the binding specificity of an antigen binding domain are called complementarity determining regions or CDR. Traditionally, CDRs of antibody variable domains have been identified as those hypervariable antibody sequences, containing residues essential for specific antigen recognition. Examples of antibodies are the immunoglobulin isotypes and their isotypic subclasses and fragments which comprise an antigen binding domain. Examples of such fragments are (I) the Fab fragment comprising the VL, VH, CL and CH1 domains; (II) the Fd fragment comprising the VH and CH1 domains; (III) the Fv fragment comprising the VL and VH domains; (IV) the dAb fragment (Ward et al. (1989) Nature, 9, 341, 544-546) which comprises a VH domain; (V) isolated CDR regions; (VI) F(ab')2 fragments, a bivalent fragment comprising two linked Fab fragments; (VII) scFv fragments, wherein a VH domain and a VL domain are linked by a polypeptide linker (Bird et al. (1988) Science, 242, 423-426; Huston et al. (1988) Proc. Natl. Acad. Sci. USA, 85, 5879-83); (VIII) bispecific scFv dimers (PTC/US92/09965); (IX); and (IX) diabodies, multivalent or multispecific fragments (WO94/13804; Holliger et al. (1993) Proc. Natl. Acad. Sci. USA, 90, 6444-48). Other forms of bispecific antibodies are e.g. single-chain CRABs described by Neri et al. ((1995) J. Mol. Biol., 246, 367-73). State of the art techniques allow easily by use of recombinant DNA technology to graft the antigen binding domain or the complementary determining regions (CDRs) of one antibody or fragment, e.g. from a Fab antibody, into the framework of another antibody format to produce other antibodies or fragments, e.g. scFv or IgG, which retain the specificity of the original antibody. See for instance amongst others EP-A-184187, GB 2188638A, or EP-A-239400.

In addition to their known uses in diagnostics, antibodies and fragments thereof have been shown to be useful as therapeutic agents. For example, immunotherapy, or the use of antibodies for therapeutic purposes has been used in recent years to treat cancer. Passive immunotherapy involves the use of monoclonal antibodies in cancer treatments. See for example, Cancer: Principles and Practice of Oncology, 6.sup.th Edition (2001) Chapt. 20 pp. 495-508. These antibodies can have inherent therapeutic biological activity both by direct inhibition of tumor cell growth or survival and by their ability to recruit the natural cell killing activity of the body's immune system. These agents can be administered alone or in conjunction with radiation or chemotherapeutic agents.

In another preferred embodiment of the compound of general formula (II) according to the present invention at least one of T1 and T2 is an aptamer oligonucleotide.

In a further preferred embodiment of the compound of general formula (II) according to the present invention at least one of T1 and T2 is a bivalent aptamer oligonucleotide.

The term "bivalent aptamer oligonucleotide" refers to compounds containing two pharmacophores connected through a spacer. They should be useful as tools to probe the structural organization and significance of certain targets. Binding experiments have indicated that bivalent aptamers may approach the affinity and the dissociation rate of bivalent antibodies, and preferentially may recognize cellular compared to soluble targets, which is a potentially useful distinction in vivo.

In an even more preferred embodiment of the compound of general formula (II) according to the present invention at least one of T1 and T2 is an aptamer oligonucleotide which comprises DNA or RNA or combinations thereof.

The aptamer of the nucleic acid of the invention can be comprised entirely of RNA. In other embodiments of the invention, however, the aptamer can instead be comprised entirely of DNA, or partially of DNA, or partially of other nucleotide analogs.

Aptamers are typically developed to bind particular ligands by employing known *in vivo* or *in vitro* (most typically, *in vitro*) selection techniques known as SELEX (Systematic Evolution of Ligands by Exponential Enrichment). Methods of making aptamers are described in, for example, Ellington and Szostak, (1990) Nature 346, 818; Tuerk and Gold, (1990) Science 249, 505; U.S. Pat. No. 5,582,981, PCT Publication No. WO 00/20040; U.S. Pat. No. 5,270,163; Lorsch and Szostak, (1994) Biochemistry, 33, 973; Mannironi et al., (1997) Biochemistry 36, 9726; Blind, (1999) Proc. Natl. Acad. Sci. USA 96, 3606-3610; Huizenga and Szostak, (1995) Biochemistry, 34, 656-665, PCT Publication Nos. WO 99/54506; WO 99/27133,; WO 97/42317 and U.S. Pat. No. 5,756,291.

Generally, in their most basic form, in vitro selection techniques for identifying RNA aptamers involve first preparing a large pool of DNA molecules of the desired length that contain at least some region that is randomized or mutagenized. For instance, a common oligonucleotide pool for aptamer selection might contain a region of 20-100 randomized nucleotides flanked on both ends by an about 15-25 nucleotide long region of defined sequence useful for the binding of PCR primers. The oligonucleotide pool is amplified using standard PCR techniques. The DNA pool is then transcribed in vitro. The RNA transcripts are then subjected to affinity chromatography. The transcripts are most typically passed through a column or contacted with magnetic beads or the like on which the target ligand has been immobilized. RNA molecules in the pool which bind to the ligand are retained on the column or bead, while nonbinding sequences are washed away. The RNA molecules which bind the ligand are then reverse transcribed and amplified again by PCR (usually after elution). The selected pool sequences are then put through another round of the same type of selection. Typically, the pool sequences are put through a total of about three to ten iterative rounds of the selection procedure. The cDNA is then amplified, cloned, and sequenced using standard procedures to identify the sequence of the RNA molecules which are capable of acting as aptamers for the target ligand.

For use in the present invention, the aptamer is preferably selected for ligand binding in the presence of salt concentrations and temperatures which mimic normal physiological conditions.

Once an aptamer sequence has been successfully identified, the aptamer may be further optimized by performing additional rounds of selection starting from a pool of oligonucleotides comprising the mutagenized aptamer sequence.

One can generally choose a suitable ligand without reference to whether an aptamer is yet available. In most cases, an aptamer can be obtained which binds the small, organic molecule of choice by someone of ordinary skill in the art. The unique nature of the in vitro selection process allows for the isolation of a suitable aptamer that binds a desired ligand despite a complete dearth of prior knowledge as to what type of structure might bind the desired ligand.

In another more preferred embodiment of the compound of general formula (II) according to the present invention at least one of T₁ and T₂ is an aptamer oligonucleotide conjugated to an N₃S chelator.

In a further more preferred embodiment of the compound of general formula (II) according to the present invention at least one of T₁ and T₂ is an aptamer oligonucleotide selected from the group of aptamer oligonucleotides that bind to tenascin-C.

The term "tenascin-C" refers to a disulfide-bonded hexamer composed of subunits with molecular weights in the range of 120-300 kD, depending on the expression of different isoforms in different species. The subunits contain epidermal growth factor (EGF)-like and fibronectin-like repeats commensurate with the growth-promoting properties of tenascin-C (Swindle, C. S., Tran, K. T., Johnson, T. D., Banerjee, P., Mayes, A. M., Griffith, L., and Wells, A. (2001) Epidermal growth factor (EGF)-like repeats of human tenascin-C as ligands for EGF receptor. J. Cell. Biol., 154, 459-68.). Tenascin-C, the proteotypical tenascin, was originally isolated from embryonic tissues (Chiquet-Ehrismann, R., Mackie, E. J., Pearson, C. A., and Sakakura, T. (1986), Tenascin: an extracellular matrix protein involved in tissue interactions during fetal development and oncogenesis. Cell. 47, 131-139; Erickson, H. P. and Bourdon, M. A. (1989) Tenascin: an extracellular matrix protein prominent in specialized embryonic tissues and tumors. Annu. Rev. Cell Biol., 5, 71-92). It is the most widely distributed tenascin and is typically seen only at sites of epithelial-mesenchymal interaction in mature tissues. It is involved in various cellular functions, such as growth promotion, hemagglutination, immunosuppression of T-cells, promotion of angiogenesis and chondrogenesis. It also has an anti-adhesive effect on many cell types (Fischer, A., Cavazzana-Calvo, M., De Saint Basile, G., De Villartay, J. P., Di Santo, J. P., Hivroz, C., Rieux-Laucat, F., and Le Deist, F. (1997) Naturally occurring primary deficiencies of the immune system. Annu. Rev. Immunol., 15, 93-124.).

In another more preferred embodiment of the compound of general formula (II) according to the present invention at least one of T₁ and T₂ is an aptamer oligonucleotide selected from TTA1 and TTA3.

"TTA1 and TTA3" refers to Tenascin-C aptamers. TTA1 is a 39mer oligonucleotide (molecular weight of 13.4 kDa) that structurally recognizes human Tenascin-C and binds to it with high affinity (K_{d} of 5 × 10⁻⁹ M) (Hicke, B. J., Marion, C., Chang, Y.-F., Gould, T., Lynott, C. K., Parma, D., Schmidt, P. G. and Warren, S. (2001) Tenascin-C aptamers are generated using tumor cells and purified protein. J. Biol. Chem., 276, 48644-48654.). Tenascin-C is a hexameric protein found in the extracellular matrix that plays an important role in tumorigenesis, embryogenesis and wound healing (Erickson, H. P. and Bourdon, M. A. (1989) Tenascin: an extracellular matrix protein prominent in specialized embryonic tissues and tumors. Annu. Rev. Cell Biol., 5, 71-92). Based on this feature, TTA1 labeled with technetium-99m (Tc-99m) is a promising candidate for imaging of tumors expressing Tenascin-C. The TTA1 aptamer is currently in clinical trials. Similar application applies for TTA3.

A particularly preferred compound according to the present invention is

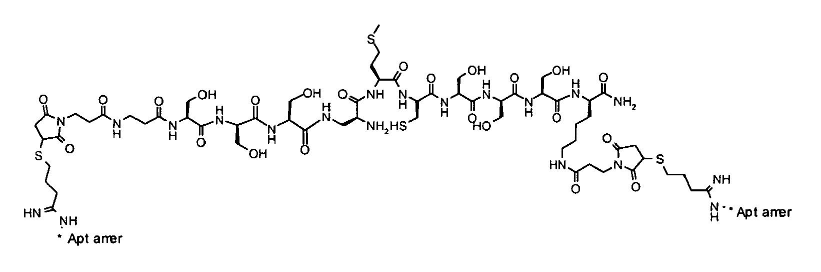

In another preferred embodiment of the compound of general formula (II) according to the present invention, the compound further comprises a metal ion.

In another more preferred embodiment of the compound of general formula (II) according to the present invention the compound comprises a metal ion which is a radioactive isotope suitable for radio therapy or for diagnostic imaging.

The person of skill in the art will recognize that the compound of the present invention can be labeled with covalently coupled radioactive isotopes, including halogens and there in particular ¹⁸F, ¹²⁵I, ¹³¹I, ¹²³I, ⁷⁶Br , ⁷⁷Br, ⁸⁰Br, ⁸²Br and ²¹¹At. One or more of these radioisotopes are coupled through a covalent bond to the compounds of the present invention. In principal the radioisotope can be coupled to any moiety within the compound, which is capable of forming a bond to the respective radioisotope.

The term "radio therapy" refers to a therapeutic treatment for many types of cancers and is delivered in various ways, depending on the disease, its location, and its stage. Such therapy may include different isotopes, for example caesium, iridium, iodine, or cobalt as the radiation source for external irradiation. The radiation therapy may be whole body irradiation, or may be directed locally to a specific site or tissue in or on the body. Typically, radiation therapy is administered in pulses over a period of time from about 1 to about 2 weeks. The radiation therapy may, however, be administered over longer periods of time. Optionally, the radiation therapy may be administered as a single dose or as multiple, sequential doses. Examples of radiation therapies include conformal radiation therapy, coronary artery brachytherapy, fast neutron radiotherapy, intensity modulated radiotherapy (IMRT), interoperative radiotherapy, interstitial brachytherapy, interstitial breast brachytherapy, organ preservation therapy, and steriotactic radiosurgery. Radiotherapy can also be performed by systemic administration of a radiopharmaceutical disclosed by this invention. The use of therapeutic radiopharmaceuticals is also encompassed by the invention.

In a further preferred embodiment of the compound of general formula (II) according to the present invention the compound comprises a metal ion, wherein the metal ion is selected from the group of ¹⁰⁷Ag, ¹⁰⁹Ag, ¹¹¹Ag, ⁷⁵As, ⁷⁷As, ¹⁹⁷Au, ¹⁹⁸Au, ¹⁹⁹Au, ²⁰⁹Bi, ²¹²Bi, ²¹³Bi, ⁶³Cu, ⁶⁵Cu, ⁶⁷CU, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy, ¹⁶²Er, ¹⁶⁴Er, ¹⁶⁶Er, ¹⁶⁷Er, ¹⁶⁸Er, ¹⁶⁹Er, ¹⁷⁰Er, ¹⁷¹Er, ¹⁷²Er, ⁶⁷Ga, ⁶⁸Ga, ⁶⁹Ga, ⁷¹Ga, ¹⁵⁴Gd, ¹⁵⁵Gd, ¹⁵⁶Gd, ¹⁵⁷Gd, ¹⁵⁸Gd, ¹⁵⁹Gd, ¹⁶⁰Gd, ¹⁶⁵Ho, ¹⁶⁶Ho, ¹²³I, ¹²⁷I, ¹²⁹I, ¹³¹I, ¹¹¹In, ¹¹³In, ¹⁹¹Ir, ¹⁹²Ir ¹⁹³Ir, ¹⁷⁵Lu, ¹⁷⁷Lu, ⁹²Mo, ⁹⁴Mo, ⁹⁵Mo, ⁹⁶Mo, ⁹⁷Mo, ⁹⁸Mo, ⁹⁹Mo, ¹⁰⁰Mo, ³¹P, ³²P, ³³p, ¹⁰²Pd, ¹⁰³Pd, ¹⁰⁴Pd, ¹⁰⁵Pd, ¹⁰⁶Pd, ¹⁰⁸Pd, ¹⁰⁹Pd, ¹¹⁰Pd, ¹⁴¹pr, ¹⁴²Pr, ¹⁴³Pr, ¹⁸⁵Re, ¹⁸⁶Re, ¹⁸⁸Re, ¹⁰³Rh, ¹⁰⁵Rh, ⁴⁵Sc, ⁴⁷Sc, ¹⁴⁴Sm, ¹⁴⁹Sm, ¹⁵⁰Sm, ¹⁵²Sm, ¹⁵³Sm, ¹⁵⁴Sm, ¹¹²Sn, ¹¹⁴Sn, ¹¹⁵Sn, ¹¹⁶Sn, ¹¹⁷Sn, ¹¹⁷Sn, ¹¹⁸Sn, ¹¹⁹Sn, ¹²⁰Sn, ¹²¹Sn, ¹²²Sn, ¹²⁴Sn, ⁸⁴Sr, ⁸⁶Sr, ⁸⁷Sr, ⁸⁸Sr, ⁸⁹Sr, ¹⁵⁹Tb, ¹⁶¹Tb, ^{94m}Tc, ⁹⁷Tc, ⁹⁸Tc, ⁹⁹Tc, ^{99m}Tc,¹²⁰Te,¹²²Te, ¹²⁴Te, ¹²⁵Te, ¹²⁶Te, ¹²⁷Te, ¹²⁸Te, ¹⁶⁹Tm, ¹⁷²Tm,⁸⁶Y, ⁸⁹Y, ⁹⁰Y, ¹⁶⁸Yb, ¹⁶⁹Yb, ¹⁷⁰Yb, ¹⁷¹Yb, ¹⁷²Yb,¹⁷³Yb, ¹⁷⁴Yb, ¹⁷⁵Yb, and ¹⁷⁶Yb.

In an even further preferred embodiment of the compound of general formula (II) according to the present invention the compound comprises a metal ion, wherein the metal ion is selected from the group consisting of ^{99m}Tc, ⁸⁶Y, ⁶⁸Ga,¹¹¹In, ¹⁸⁶Re, ¹⁸⁸Re, ⁹⁰Y, ⁶⁴Cu, ⁶⁷Ga, ¹⁷⁷Lu.

Though in this context any metal ion may be bound to the compound, however, preferred metals are γ-emitting radionuclides such as ¹¹¹In, ⁶⁷Ga and ^{99m}Tc, β-emitting radionuclides such as ⁹⁰Y, ¹⁸⁶Re, ¹⁸⁸Re and ¹⁷⁷Lu or positrone-emitting radionuclides such as ⁶⁸Ga and ⁸⁶Y.

In yet another preferred aspect, the present invention relates to a kit for preparing a radiopharmaceutical preparation, said kit comprising a vial comprising a quantity of the compound according to the invention, Preferably, the kit comprises a sealed vial containing a predetermined quantity of the compound according to the present invention.

In a further preferred aspect, the present invention provides a method for producing a compound of general formula (I) according to the present invention, comprising synthesis in an automated peptide synthesizer.

Chemical peptide synthesis techniques (including manual and automated techniques) which are suitable for directly synthesizing the compound are also well-known to those of ordinary skill in the art. For example, the compound can be synthesized de novo using conventional solid phase synthesis methods. In such methods, the peptide chain is prepared by a series of coupling reactions in which the constituent amino acids are added to the growing peptide chain in the desired sequence. The use of various N-protecting groups, e.g., the carbobenzyloxy group or the t-butyloxycarbonyl group; various coupling reagents e.g., dicyclohexylcarbodiimide or carbonyldimidazole; various active esters, e.g., esters of N-hydroxyphthalimide or N-hydroxysuccinimide; and the various cleavage reagents, e.g., trifluoroactetic acid (TFA), HCl in dioxane, boron tris-(trifluoracetate) and cyanogen bromide; and reaction in solution with isolation and purification of intermediates are well-known to those of ordinary skill in the art. A preferred chemical peptide synthesis method follows conventional Merrifield solid phase procedures well known to those skilled in the art. Additional information about solid phase synthesis procedures can be had by reference to Steward and Young, Solid Phase Peptide Synthesis, W. H. Freeman & Co., San Francisco, 1969; the review chapter by Merrifield in Advances in Enzymology 32, 221-296, (Nold, F. F., ed.), Interscience Publishers, New York, 1969; and Erickson and Merrifield (1990), The Proteins 2, 61-64, the entire disclosures of which are herein incorporated by reference.

In a yet further preferred aspect, the present invention provides a method of producing a compound of general formula (II) according to the present invention, comprising attaching a compound of general formula (I) according to the present invention through the maleimide group to a targeting moiety.

In another preferred embodiment of the method of producing a compound of general formula (II) according to the present invention, comprising attaching a compound of general formula (I) according to the present invention through the maleimide group to a targeting moiety, the targeting moiety is selected from the group of an aptamer oligonucleotide, a peptide, an antibody or a fragment thereof.

In a particular preferred embodiment of the method according to the present invention, the targeting moiety is an aptamer oligonucleotide.

In a further preferred aspect thereof, the present invention relates to a compound according to the present invention for use in therapy and/or diagnosis.

In a further preferred aspect thereof, the present invention relates to the use of a compound according to the present invention for the manufacture of a medicament for diagnosis or therapy of a proliferative disease.

In another aspect, the present invention relates to the use according the present invention, wherein the proliferative disease is selected from the group consisting of a tumor, a precancerosis, a dysplasia and a metaplasia.

In a further aspect, the present invention concerns the use according to the present invention, wherein the tumor is a primory tumor or a metastasis.

In a further aspect thereof, the present invention relates to the use according to the present invention, wherein the tumor is a malignoma of the gastrointestinal or colorectal tract, liver, pancreas, kidney, bladder, thyroid, prostate, endometrium, ovary, testes, melanoma, dysplastic oral mucosa, invasive oral cancer, small cell or non-small cell lung carcinoma; a mammary tumor, including hormone-dependent breast cancer, hormone independent breast cancers, transitional and squamous cell cancers, neurological malignancy including neuroblastoma, gliomas, astrocytomas, osteosarcoma, meningioma; soft tissue sarcoma; hemangioama and an endocrinological tumor, including pituitary adenoma, pheochromocytoma, paraganglioma, a haematological malignancy including lymphoma and leukemia or the metastasis originates from one of above mentioned tumors.

In another preferred embodiment the present invention provides methods for treating patients comprising administering to said patient in need an effective amount of a compound according to the present invention.

In another preferred embodiment the present invention provides methods according to the present invention, wherein said patient has a proliferative disease.

In another preferred aspect thereof, the present invention provides methods for diagnosing patients utilizing the compounds according to the present invention.

In a further preferred embodiment, the present invention relates to methods for diagnosing patients utilizing the compounds according to the present invention, wherein the patients have a proliferative disease.

In one further preferred embodiment, the present invention concerns a pharmaceutical composition comprising a compound of general formula (I) and (II) according to the present invention and a pharmaceutically applicable carrier and/or excipient.

### BRIEF DESCRIPTION OF THE FIGURES

Figure 1 shows the synthesis of peptides. a) piperidine, DMF, b) hydrazine, DMF, c) N-succinimidyl-maleimido-propionate, d) TFA cleavage
Figure 2 shows the chelating moieties Dpr-Gly-Cys (a) and Dpr-Met-Cys (b).
Figure 3 shows gel electrophoresis analysis of the aptamer dimer reaction mixture and the starting material.

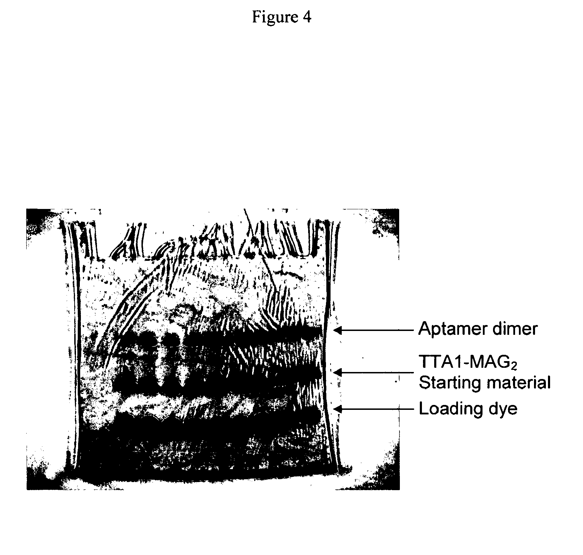

Figure 4 shows an example of a gel used for gel electrophoresis.
Figure 5 shows an HPLC control of purification success.
Figure 6 shows a PAGE of the purified dimer (22).
Figure 7 shows mass spectra of (22).
Figure 8 shows plasma stabilities of Tc-99m labeled dimers in human plasma.
Figure 9 shows the dynamic organ distribution of (2A), (22A) and (23A).
Figure 10 shows the organ distribution of (2A), (22A) and (23A) in U251 tumor bearing NMRI nude mice.
Figure 11 shows the dynamic imaging study of (2A) and (23A) in nude NMRI mice. A: Dynamic image, B: Blood clearance of (2A) in dependence of time, C: Blood clearance of (23A) in dependence of time.

### EXAMPLES

The invention is illustrated by the following examples. The examples merely show selected embodiments of the invention and are not limiting for the invention. The scope of the invention is defined only by the appended claims.

### Example 1. Synthesis of peptides

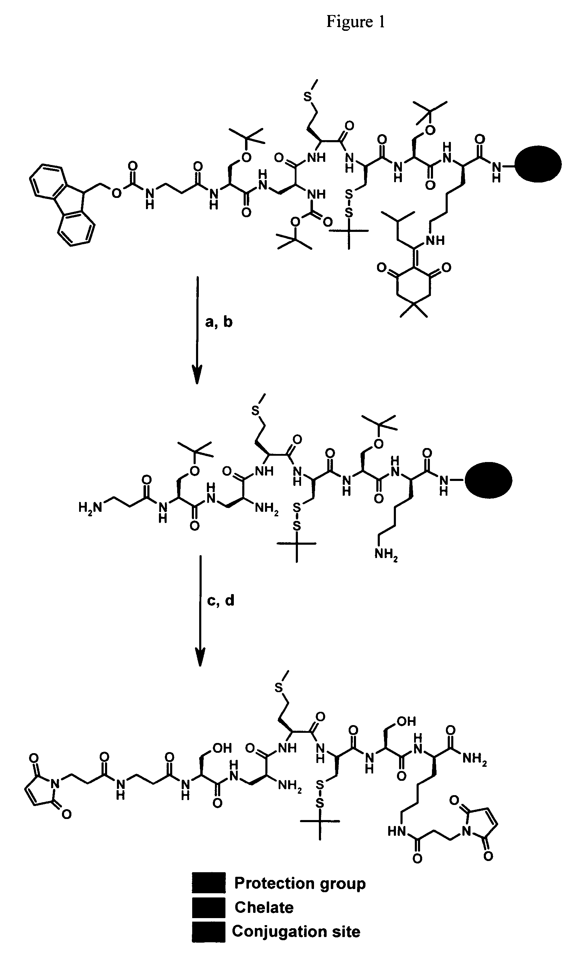

The peptide sequences were synthesized, see Figure 1, in an automated peptide synthesizer (Advanced ChemTech, 396 multiple well peptide synthesizer) using a standard coupling procedure. 800 µl of a 0.5 M amino acid solution in DMF, 800 µl of a 0.5 M 6-Chloro-1-Hydroxybenzotriazole (HOBt) and O-benzotriazole, N,N,N',N'-tetramethyl-uronium-hexafluoro-phosphate (HBTU) solution in DMF and 800 µl of a 1 M diisopropyethylamine (DIPEA) solution were added to a well. The reaction mixture in the reaction block was mixed for 45 minutes at 800 rpm, afterwards the wells were emptied and washed with DMF before a new coupling cycle was started. After the last coupling the resin was washed with dichloromethane (CH₂Cl₂), tetrahydrofurane (THF) and DMF.

The Fmoc (9-fluorenylmethyloxycarbonyl) protection groups were cleaved with 50 % piperidine in DMF and subsequently the ivDde ([1-(4,4-dimethyl-2,6-dioxocyclohexylidene)3-methyl-butyl]) protection group was cleaved using 2 % hydrazine in DMF to leave the free amine groups that are conjugated to maleimide building blocks. 0.6 mmol N-succinimidyl 3-maleimidopropionate and 0.6 mmol diisopropyl ethyl amine in 2 ml DMF were added to 0.2 mmol of the resin. The reaction mixture was stirred for one hour, afterwards the solvent was removed and the resin washed extensively with DMF. A ninhydrin standard test was employed to indicate unreacted free amine groups. Subsequently, the peptides were cleaved from the resin. During the cleavage reaction in 85 % TFA, 5 % phenol, 5 % water, 5 % triisopropansilane the tertiary butyl protection groups were eliminated as well. The cleaved peptides still contain one protection group, S-^{t}Bu, which protects the SH-group in cysteine.

The peptides P1-P6, see Table 1, were prepared using automated peptide synthesis on an Apex 396 multiple well peptide synthesizer (Advanced ChemTech, Louisville, KY). Ten wells of a 40-well block were utilized with 0.1 mmol of resin in each well.

### Standard coupling procedure:

0.8 ml of a 0.5 M amino acid solution in DMF, 0.8 ml of a 0.5 M 6-Chloro-l-Hydroxybenzotriazole (HOBt) and O-Benzotriazole,N,N,N',N'-tetramethyl-uronium-hexafluoro-phosphate (HBTU) solution in DMF, and 0.8 ml of a 1 M diisopropyethylamine (DIPEA) solution were added to a well of the reaction block. The reaction block containing the reaction mixtures was mixed at 800 rpm for 45 minutes. Afterwards, the wells were emptied with nitrogen and washed with DMF. The coupling was repeated until the desired peptide sequence was finished and subsequently, the resin was washed with CH₂Cl₂, THF, and DMF.

### Standard Fmoc deprotection:

1.8 ml of DMF was transferred to the well followed by 1.2 ml 50% piperidine in DMF. The reaction block was mixed at 800 rpm for 5 minutes then emptied and the wells were washed with DMF. The deprotection was repeated, this time for 20 minutes. The resin was then washed with DMF, CH₂Cl₂, and DMF.

### Deprotection of iv-dde groups:

2 % hydrazine in DMF was added to the reaction vial with the resin. After 10 minutes the hydrazine solution was removed. The deprotection reaction was repeated twice. Afterwards the resin was washed five times with DMF. A ninhydrin standard test was performed to proof the deprotection success.

### Addition of Maleimide-group:

The reaction solution containing 160 mg (0.6 mmol) N-Succinimidyl 3-Maleimidopropionate and 130 mg (0.6 mmol) diisopropyl ethyl amine in 2 ml DMF was added to the resin. After one hour the solvent was removed and the resin washed extensively with DMF and dichloromethane. A ninhydrin standard test was used to indicate unreacted free amine groups.

### Cleavage of the resin:

All cleavages carried out using a mixture of 85 % trifluoroacetic acid, 5 % phenol, 5 % triisopropylsilane, and 5 % water. After five hours, the resin was removed by filtration, and washed with a small quantity of CH₂Cl₂. The peptide was then precipitated using cold t-butyl methyl ether (minimum ratio of 1:5 of eluent to ether), and cooled in anice-bath for 30 minutes. Afterwards, it was centrifuged for 5-10 minutes at 3000 rpm. The supernatant was decanted and additional ether added. The product was again cooled and centrifuged and decanted. The peptide was then dried under a flow of argon, lyophilized, and analyzed by HPLC and mass spectra.

### Purification of the products:

Purification of the peptides was carried out using preparative HPLC-MS and collecting the product according to the mass. Therefore the lyophilized samples were dissolved in 3 - 4 ml of a 80 % water + 0.1 % TFA / 20 % acetonitrile + 0.1 % TFA solution before they were loaded on the column (YMC-pack ODS-AQ 150x20 mm).
The fractions containing the product were combined and lyophilized. A HPLC and a mass spectra of the final product indicated the purity.

Peptides P6 and P7 were bought on the resin and therefore the handling of these two peptides started with the Fmoc-deprotection step.

**Table 1: Sequences and abbreviation code of peptides,**

| | Sequence |
|---|---|
| P1 | Mal-βAla-Ser-Dpr-Met-Cys(tButylthiol)-Ser-Lys-Mal |
| P2 | Mal-βAla-Ser-Dpr-Gly-Cys(tButylthiol)-Ser-Lys-Mal |
| P3 | Mal-βAla-Ser-Ser-Dpr-Met-Cys(tButylthiol)-Ser-Ser-Lys-Mal |
| P4 | Mal-βAla-Ser-Ser-Dpr-Gly-Cys(tButylthiol)-Ser-Ser-Lys-Mal |
| P5 | Mal-βAla-Ser-Ser-Ser-Dpr-Met-Cys(tButylthiol)-Ser-Ser-Ser-Lys-Mal |
| P6 | Mal-βAla-D-Ser-D-Ser-D-Ser-Dpr-Met-Cys(tButylthiol)-D-Ser-D-Ser-D-Ser-Lys-Mal |
| P7 | Mal-βAla-D-Ser-D-Ser-D-Ser-Dpr-Gly-Cys(tButylthiol)-D-Ser-D-Ser-D-Ser-Lys-Mal |

Mal= Maleimide

### Example 2. Peptidic radiometal chelators for dimerization of aptamers

For the synthesis of dimeric aptamer conjugates, peptidic linkers that contain the chelating unit Dpr-Gly-Cys or Dpr-Met-Cys were synthesized in an automated peptide synthesizer. The compounds that were synthesized are listed in Table 1.

The underlying idea of aptamer dimerization in this thesis, is to synthesize peptides, containing a metal chelating unit with two adjacent linkers. Each linker carries a maleimide group for the conjugation to sulfhydryl functionalities in the aptamer. The peptidic linker should allow for a radio labeling with ^{99m}Tc which was accomplished by the introduction of a peptide sequence into the spacer that offers a N₃S donor set for the coordination of [Tc(V)O]³⁺ . The chelating site in the peptide sequence is formed by Dpr-Gly-Cys and Dpr-Met-Cys. Peptides that contain the amino acid methionine instead of glycine offer an additional side chain in the chelating sequence that could add additional stability to the metal complex. This is due to the lone electron pair of the sulfur atom in ethyl sulfanyl methane that can serve as an additional donor. The two chelating moieties are depicted in Figure 1.

This chelating sequence is abutted on two spacer sequences which can be varied in length and in hydrophilicity in order to study the influence of these factors on conjugation success and plasma stability properties *in vitro.* Under the consideration that these peptides are synthesized in an automated peptide synthesizer, several sequences can be synthesized at the same time.

In order to study the effect of the spacer length, the number of serine amino acid building blocks within the linker region was varied. Serine was chosen because of its hydrophilic nature which might have a positive effect in terms of renal elimination.

### Example 3. Peptidic Dpr-Gly-Cys and Dpr-Met-Cys for aptamer dimer formation

The bifunctional peptides (P1 - P7) were used for aptamer dimerization and radiometal complexation. TTA1 conjugated to MAG₂ was used for the dimerization of its sulfhydryl group with a maleimide group of the linker. The conjugation of the peptides to the aptamer and the subsequent reduction of the thiol protecting group is depicted in Scheme 1.

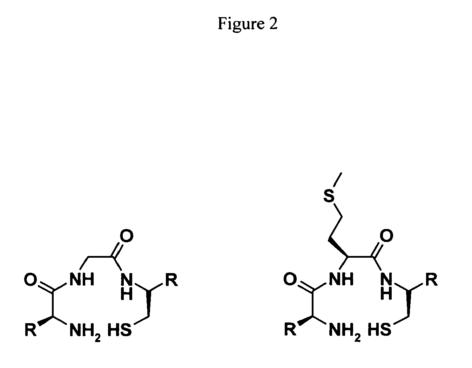

After conjugation, the reaction mixture was loaded onto a 10 x TBE-urea gel. An example of a PAGE of the starting material and the reaction mixture (TTA1-MAG₂)₂P5 is represented in Figure 2. This PAGE indicates an impurity in the starting material. Regarding the movement pattern on the gel, the impurity could be a dimer, formed by a disulfide bridge between two TTA₁-MAG₂ molecules. The ratio of starting material to dimer in the reaction mixture is shifted towards the dimer formation. This finding indicates the formation of the peptide linked aptamer dimer (TTA1-MAG₂)₂P5.

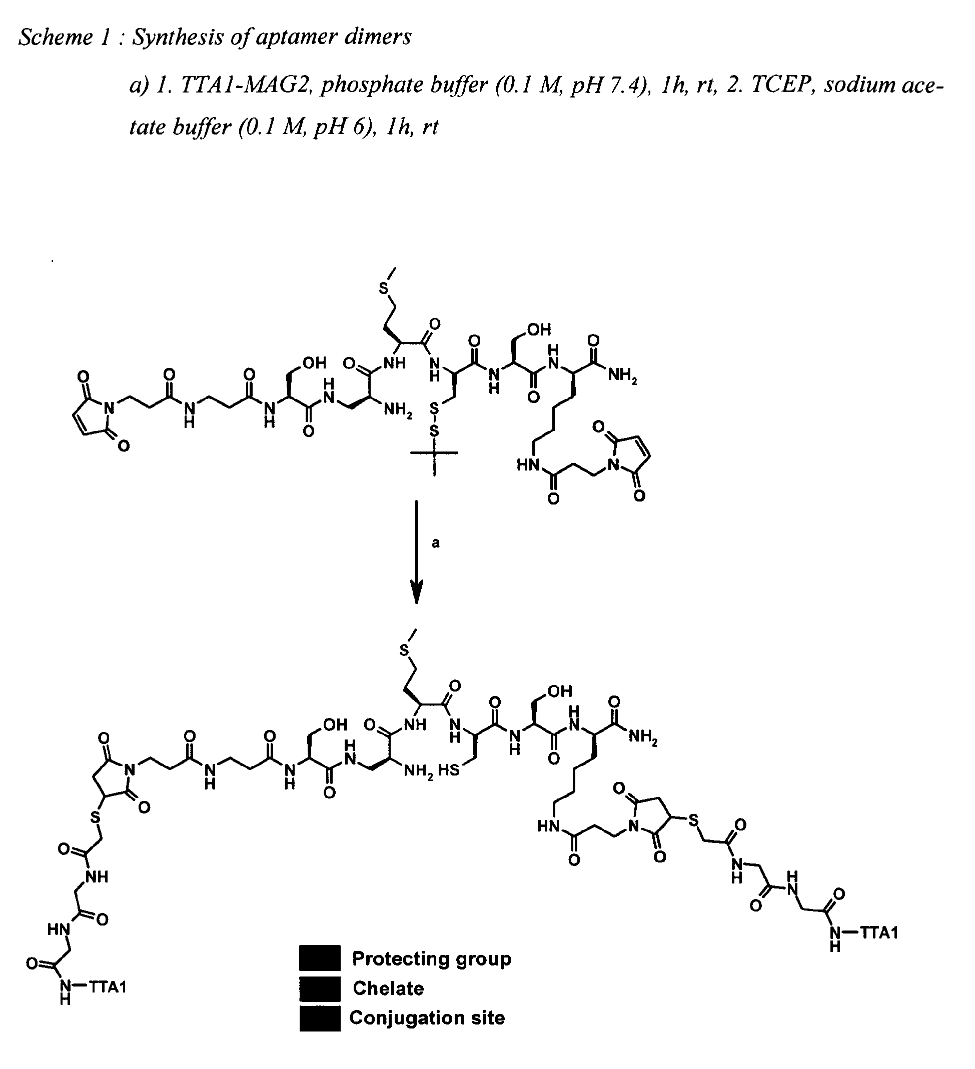

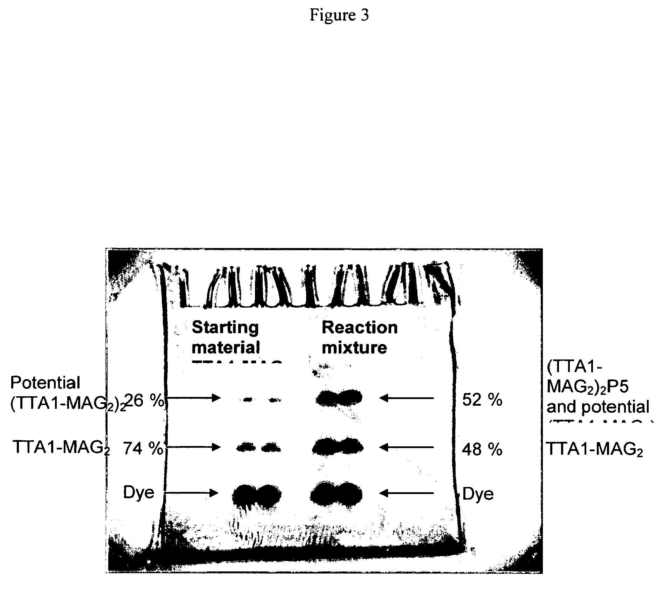

The reaction mixture was purified by preparative PAGE. After 50 minutes run time, the gel was cut into horizontal bands containing unreacted TTA1-MAG₂ and aptamer dimer. An example of a gel with a dimer and a starting material band used for gel electroelution is depicted in Figure 3. The dimer is eluted from the gel by electroelution in 1 x TBE, followed by TCEP reduction of the product. TCEP removes the ^{t}Bu-S protecting group, a prerequisite for radiometal complexation. On the other hand, it reduces also disulfide bonds in the case of the formation of TTA1-MAG₂-MAG₂-TTA1. The resulting TTA1-MAG₂ monomers were then be separated form the TTA1-MAG₂-P-MAG₂-TTA1 dimers by a second PAGE purification and gel electroelution.

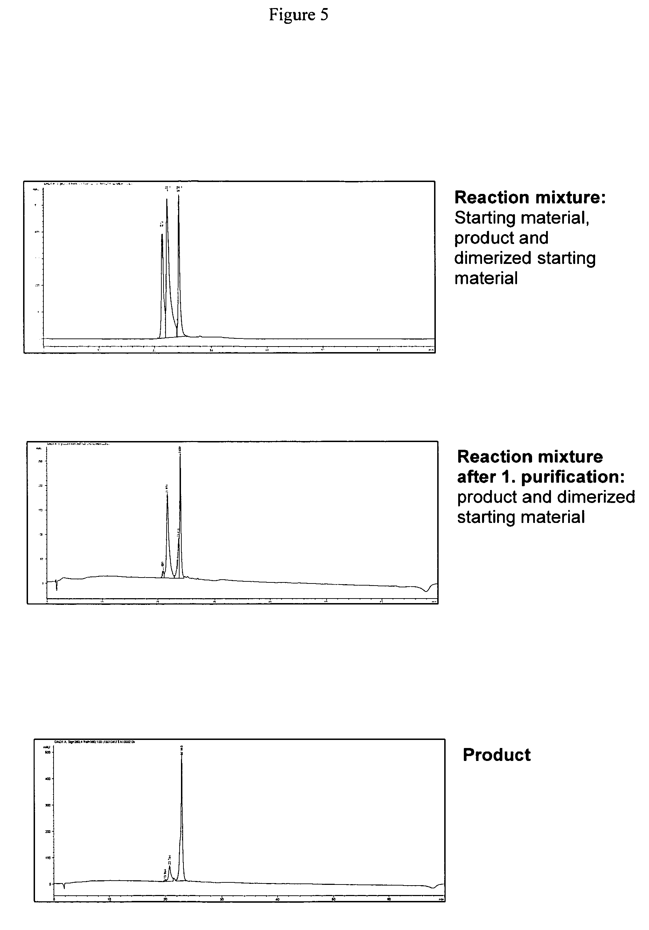

The purified dimers were characterized by HPLC, PAGE and a sample was submitted for mass spectrometry. The pursuit of the purification success was tracked by RP-HPLC. Figure 5 depicts those chromatograms.

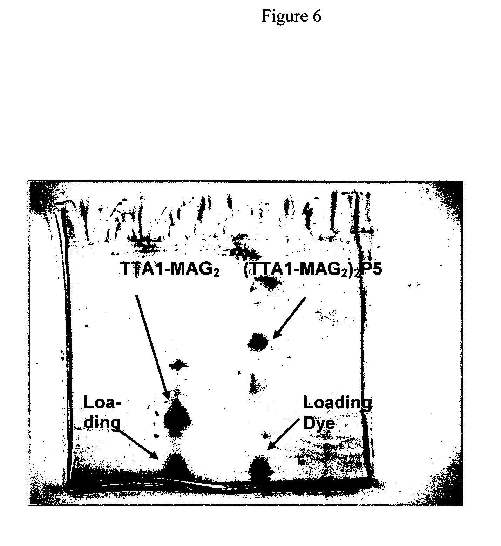

After the purification a PAGE of the product and the monomeric starting material TTA1-MAG₂ was performed to visualize the success. Figure 6 shows a PAGE of the compound (TTA1-MAG₂)₂P5 (22).

This procedure was employed to synthesize eight aptamer dimers using six different peptide spacers (P1 - P7) (Table 1) and two different aptamers, TTA1-MAG₂ (2) and TTA3-MAG₂ (13). The aptamer dimer sequences are listed in Table 2.

The expected masses of the final products ranges from 26931.31 g/mol to 28371.86 g/mol. Mass spectrometry with oligonucleotides is complicated due to their high negative surface charge and the high number of associated cations (Na⁺, K⁺). The ESI (electro spray ionization) mass analysis usually yields rather complex mass spectra with low resolution (Esmans, E. L., Broes, D., Hoes, I., Lemiere, F., and Vanhoutte, K. (1998) Liquid chromatography-mass spectrometry in nucleoside, nucleotide and modified nucleotide characterization, Journal of chromatography A, 794, 109-127; Banoub, J. H., Newton, R. P., Esmans, E., Ewing, D. F., Mackenzie, G. (2005) Recent Developments in Mass Spectrometry for the Characterization of Nucleosides, Nucleotides and Nucleic Acids, Chemical Reviews, 1-43). A purification method that ameliorate the resolution of the spectra was mandatory for the analysis of the dimeric aptamer compounds. Precipitation of the product in ethanol is a well known and an effective method for the purification (Limbach, P. A., Crain, P. F., McCloskey, J. A. (1995) Molecular Mass Measurement of Intact Ribonucleic Acids Via Electrospray Ionization Quadropole. Mass Spectrometry, 6, 27-39).

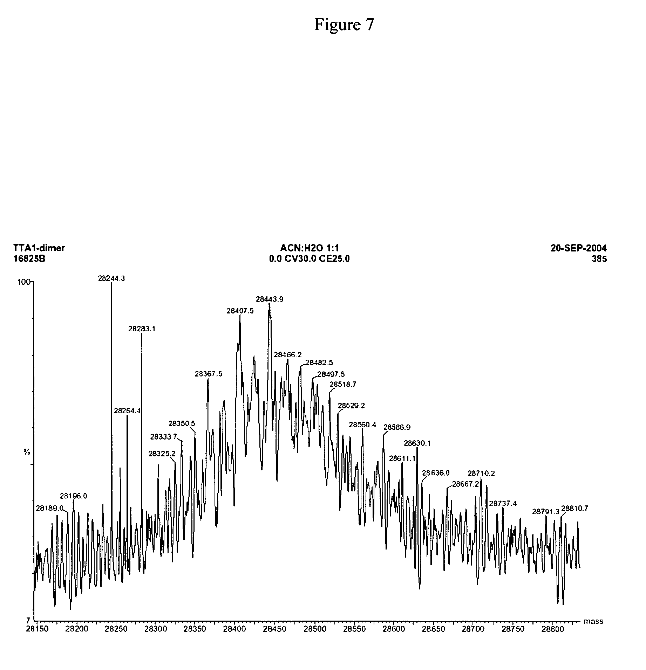

Hence, the lyophilized sample was dissolved in 10 µl of a 10 M ammonium acetate buffer. After stirring for 5 minutes, 100 µl cold ethanol was added to the sample and the mixture stored at -20°C for 3 hours to assure a complete precipitation of the oligonucleotide compound. Thereafter, the mixture was centrifuged. The residue was decantated, the pellet washed with 300 µl of a 70 % ethanolic solution and the precipitate redissolved in ammonium acetate buffer to repeat this procedure. After three purification cycles, the precipitated sample was washed and lyophilized. Figure 7 displays the mass spectra of (22).

**Table 2 : Aptamer dimers**

| Sequence | Formula | No |
|---|---|---|
| TTA1-MAG₂-Mal-βAla-Ser-Dpr-Met-Cys-Ser-Lys-Mal-MAG₂-TTA1 | (TTA1-MAG₂)₂P1 | 18 |
| TTA1-MAG₂-Mal-βAla-Ser-Dpr-Gly-Cys-Ser-Lys-Mal- MAG₂-TTA1 | (TTA1-MAG₂)₂P2 | 19 |
| TTA1-MAG₂-Mal-βAla-Ser-Ser-Dpr-Met-Cys-Ser-Ser-Lys-Mal- MAG₂-TTA1 | (TTA1-MAG₂)₂P3 | 20 |
| TTA1-MAG₂-Mal-βAla-Ser-Ser-Dpr-Gly-Cys-Ser-Ser-Lys-Mal- MAG₂-TTA1 | (TTA1-MAG₂)₂P4 | 21 |
| TTA1-MAG₂-Mal-βAla-Ser-Ser-Ser-Dpr-Met-Cys-Ser-Ser-Ser-Lys-Mal- MAG₂-TTA1 | (TTA1-MAG₂)₂P5 | 22 |
| TTA1-MAG₂-Mal-βAla-D-Ser-D-Ser-D-Ser-Dpr-Met-Cys-D-Ser-D-Ser-D-Ser-Lys-Mal- MAG₂-TTA1 | (TTA1-MAG₂)₂P6 | 23 |
| TTA1-MAG₂-Mal-βAla-D-Ser-D-Ser-D-Ser-Dpr-Gly-Cys-D-Ser-D-Ser-D-Ser-Lys-Mal- MAG₂-TTA1 | (TTA1-MAG₂)₂P7 | 24 |
| TTA3-MAG₂-Mal-βAla-Ser-Ser-Ser-Dpr-Met-Cys-Ser-Ser-Ser-Lys-MAG₂-TTA3 | (TTA3-MAG₂)₂P5 | 25 |

The conjugation reaction was slightly influenced by the length of the linker. As is shown in Table 3, there is a tendency that the length of the linker is correlated with the yield. The linker P5 and P6 have the highest conjugation yields of 28 %, while P1 gave only a yield of 22 %. Moreover, linker P5, P6 and P7 contain the highest number of serine building blocks and may help a faster renal clearance. Therefore, the stabilized aptamer TTA3 (4), was dimerized with P5 for a comparative study.

The introduction of D-amino acids into the peptide linker sequence for metabolic stabilization has been performed. Proteins are only composed of L-amino acids, therefore, replacing L-amino acids against D-amino acids provides a higher metabolic stability since most proteases cannot cleave D-amino acid residues (G Guichard, N Bankirane, G Zeder-Lutz, MHV Van Regenmortel, JP Briand, S Muller, Antigenic mimicry of natural L-peptides with retro-inverso-peptidomimetics, Proc. Natl. Acad. Sci., USA, 1994, Vol. 91, pp. 9765- 9769). Aptamers that are linked to form dimers by stabilized peptides should possess an improved *in vivo* stability. Here, stabilization was done in the peptide spacer by exchanging all L-serines against D-serine building blocks. The longest linker was chosen for the introduction of D-amino acids. Two peptides were synthesized (P6, P7), both containing the same amount of serine building blocks but hold different chelating sites (Dpr-Met-Cys (P6) and Dpr-Gly-Cys (P7)). Stabilized aptamer dimers were synthesized from TTA1-MAG₂.

**Table 3 : Results of aptamer dimer syntheses**

| Dimer | OD₂₆₀ | V (µl) | m (µg) | M (g/mol) | nmol | Yield (%) |
|---|---|---|---|---|---|---|
| (TTA1-MAG₂)₂ P1 | 0.195 | 450 | 325 | 28063.23 | 11 | 22 |
| (TTA1-MAG₂)₂P2 | 0.272 | 372 | 374 | 27989.34 | 13 | 26 |
| (TTA1-MAG₂)₂ P3 | 0.161 | 575 | 343 | 28237.34 | 12 | 24 |
| (TTA1-MAG₂)₂ P4 | 0.209 | 487 | 376 | 28163.32 | 13 | 26 |
| (TTA1-MAG₂)₂ P5 | 0.245 | 455 | 412 | 28411.51 | 14 | 28 |
| (TTA1-MAG₂)₂P6 | 0.219 | 370 | 324 | 28411.51 | 11 | 22 |
| (TTA1-MAG₂)₂ P7 | 0.303 | 375 | 412 | 28336.59 | 14 | 28 |
| (TTA3-MAG₂)₂ P5 | 0.449 | 233 | 387 | 26931.31 | 14 | 28 |

### Example 4. ^{99m}Tc(V) labeling of dimeric aptamer Dpr-Gly-Cys and Dpr-Met-Cys conjugates

Aptamer dimer conjugates (18) - (25) were labeled with ^{99m}Tc by mixing aptamer dimer conjugate (100 µg, 3.8 nmol) dissolved in a phosphate buffer (pH 7.4, 0.1 M) with a sodium tartrate solution (200 µl, 20 µmol), [^{99m}TCO₄]⁻ (1.85 GBq) and a tin(II)chloride solution (10 µl, 264 nmol). Labeling was accomplished at 95 °C for 15 minutes and a subsequent purification of the product by spin dialysis. Labeling success was verified by RP- HPLC, ITLC and PAGE. Radio chemical yield (RCY) and radio chemical purity (RCP) determined by RP-HPLC of the dimer products are depicted in Table 4. The radio labeled dimers carry the numbers (18A) - (25A).

**Table 4 : RCY and RCP of dimer products**

| | 18A | 19A | 20A | 21A | 22A | 23A | 24A | 25A |
|---|---|---|---|---|---|---|---|---|
| RCY (%) | 59 | 63 | 72 | 72 | 81 | 64 | 69 | 52 |
| RCP (%) | 95 | 95 | 97 | 96 | 100 | 100 | 97 | 100 |

With regard to the labeling yield, an influence of the length of the peptide spacer or the additional side arm, was not observed (see Example 1). Labeling of the dimers works about equally good for all linker lengths. The stability of the samples was investigated in saline solution over a period of 24 hours and was determined by RP-HPLC and PAGE. The results of this study are depicted in Table 5.

**Table 5 : Chemical stability of dimers during a period of 24 h (Dimers with the side arm are marked in red)**

| | 18A | 19A | 20A | 21A | 22A | 23A | 24A | 25A |
|---|---|---|---|---|---|---|---|---|
| Initial | 95 | 95 | 97 | 96 | 100 | 100 | 97 | 100 |
| 6 h | 95 | 95 | 97 | 97 | 93 | 92 | 88 | 89 |
| *24 h* | *84* | *71* | *90* | *86* | *86* | *86* | *78* | *87* |

### Example 5. Biological characterization of aptamer dimers

Due to the avidity effect, bivalent biomolecules, such as dimers are expected to have a slower dissociation from their target (Ringquist, S. and Parma, D. (1998) Anti-L-Selectin Oligonucleotide Ligands Recognize CD62L-Positive Leukocytes: Binding Affinity and Specificity of Univalent and Bivalent Ligands, Cytometry, 33, 394-405). Regarding chemical stability studies of radiolabeled aptamer dimer conjugates in solution, an eminent correlation between the length of the linker and the stability of the compound was observed. On top of these results, the conjugation yields of these linkers to the aptamer was higher for the longer linkers. Therefore, the radiolabeled dimer with the longest linker (22A) was chosen as an example for the dimers for the binding studies. Binding affinity of (22A) to human tenascin-C was measured in a competition binding assay against the lead compound. The EC₅₀ value for (TTA1-MAG₂)₂P5 is 3.6 nM and therefore, the binding of the dimer is slightly better than the binding of the monomer (IC₅₀ = 5.6 nM). The modified aptamer TTA3 was also dimerized using the peptide linker P5 (25). This compound has a binding affinity to human tenascin-C of 18.64 nM.

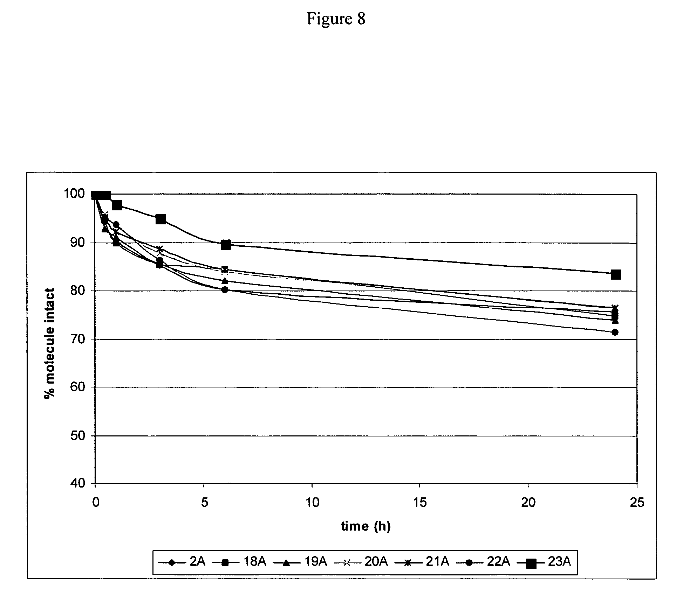

All radiolabeled aptamer dimer conjugates were tested for their stability in human blood plasma and compared to each other. Figure 8 depicts the plasma stabilities of the different dimers (18A) - (25A) in comparison to the lead compound. After an incubation time of 6 hours, the stabilities for the non-stabilized dimers (18A) - (22A) and (25A) were 80 - 85 %, a stability that is comparable to the lead compound. A remarkable increase of the stability is observed for the stabilized aptamers (23A) and (24A). Exchanging L-serine against D-serine lead to a stability of 90 % six hours after incubation in plasma. This result is in accordance with the literature, since it has been found before that D-amino acids have a prolonged stability in human blood plasma due to the resistance to lysosomal proteases (Hong, S. Y., Oh, J. E., and Lee, K. H. (1999) Effect of D-Amino Acid Substitution of the Stability, the Secondary Structure, and the Activity of Membrane-Active Peptide, Biochemical Pharmacology, 58, 1775-1780; Ehrenreich, B. A., and Cohn, Z. A. (1969) The fate of peptides pinocytosed by macrophages in vitro, J. Exp. Med., 129, 227-243).

### Aptamer dimer compounds

To investigate if the stabilization as well as the avidity effect of the radiolabeled dimeric compounds has an impact on the pharmacological behavior, an organ distribution study with (22A) and (23A) was performed. In Table 6 the results of the bio distribution in U251 NMRI tumor bearing mice are depicted. The bio distribution of (22A) does not represent the trend of an increased binding, the tumor uptake is comparable to the tumor uptake of the monomer. This is accompanied by an increased kidney and liver uptake and a fast blood clearance. Moreover, the expected increase in elimination through the kidney due to the long hydrophilic linkers can not be observed. Observing neither an increased tumor uptake nor a prolonged blood retention time could signify that the aptamer dimer conjugate (TTA1-MAG₂)₂P5 is not stable *in vivo* and gets degraded into a monomer rather quickly. An approach to circumvent this problem could be the inauguration of D-amino acids into the linker.

**Table 6 : Bio distribution in U251 tumor bearing NMRI nude mice, 5h p. i., %ID / g**

| | Tumor (%ID/g) | T/B (%ID/g) | T/K (%ID/g) | T/L (%ID/g) | Urine (%ID) |
|---|---|---|---|---|---|
| TTA1-MAG₂ | 0.41 | 4.56 | 1.86 | 0.90 | 31.00 |
| (TTA1-MAG₂)₂P5 | 0.38 | 4.75 | 0.37 | 0.10 | 33.95 |
| (TTA1-MAG₂)₂P6 | 0.8 | 10 | 0.38 | 0.14 | 22.36 |

| | | | | | |
|---|---|---|---|---|---|
| T: Tumor, B: Blood, K: Kidney, L: Liver | | | | | |

The organ distribution study of the stabilized aptamer dimer (23A) revealed an increased tumor uptake in comparison to the lead compound. This could be a result of the avidity effect. These results support the finding of various research groups investigating pharmacological differences of single chain Fv fragments (scFv) monomers versus scFv multimers (Hudson, P. J., and Kortt, A. A. (1999) High avidity scFv multimers; diabodies and triabodies, Journal of Immunological Methods, 231, 177- 189; Tahtis, K., Lee, F. T., Smyth, F. E., Power, B. E. Renner, C., Brechbiel, M. W., Old, L. J., Hudson, P. J., and Scott, A. M. (2001) Biodistribution Properties of 111Indium-labeled C-Functionalized trans-Cyclohexyl Diethylenetriaminepentaacetic Acid Humanized 3S193 Diabody and F(ab')2 Constructs in a Breast Carcinoma Xenograft Model, Clinical Cancer Research, 7, 1061- 1072). Due to the avidity effect functional binding to the antigen was increased in the case of multimeric scFvs. Furthermore, the longer blood resistance time of higher molecular weight antibody fragments allows for a longer tumor penetration time and which leads to a higher tumor uptake (Wu, A. M., Chen, W., Raubitschek, A., Williams, L. E., Neumaier, M., Fischer, R., Hu, S. Z., Odom-Maryon, T., Wong, J. Y. C., and Shively, J. E. (1996) Tumor localization of anti-CEA single-chain Fvs: improved targeting by non-covalent dimers, Immunotechnology, 2, 21 - 36).

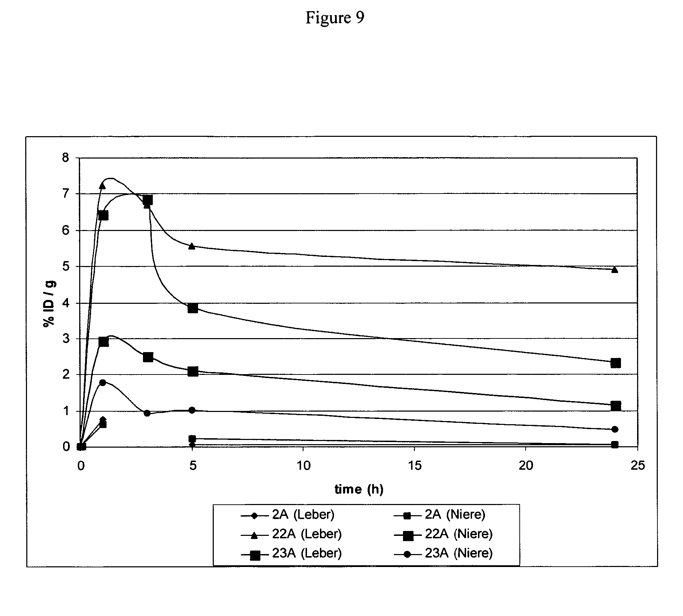

With the increased tumor uptake the tumor to blood ratio is also increased. A serious drawback to the dimer approach however, is their high and persistent uptake in the liver as well as in the kidneys which was also observed for (22A). This is an unexpected phenomenon because the urine excretion should be enhanced given that the hydrophilic linkers should have a tendency to direct the excretion of the molecule towards a fast kidney elimination. The excretion pattern of the dimers from the liver as well as from the kidney in comparison to the lead compound are depicted in Figure 9. This graphic clearly displays a correlation of the accumulation in liver and kidneys with the molecule whereas the stabilized dimer (23A) has the highest accumulation.

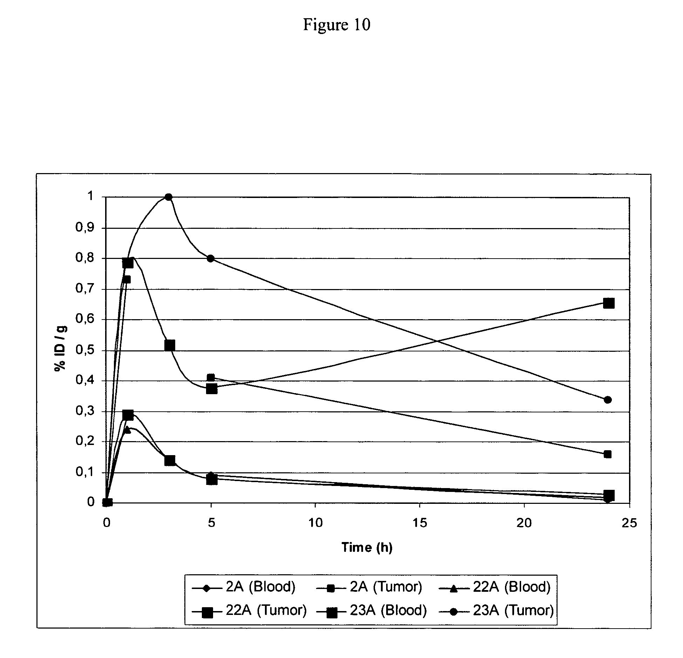

(2A) and (22A) both show a similar pharmacodynamic behavior in terms of blood and tumor clearance. The stabilized dimer has an enhanced tumor uptake and with it an ameliorated tumor to blood ratio in comparison to the lead compound. This is depicted in Figure 10. A longer blood retention time *in vivo* that is observed for multimeric scFv (~ 60 - 90 kDa) in comparison to scFv monomers (~ 30 kDa) (Todorovska, A., Roovers, R. C., Dolezal, O., Kortt, A. A., Hoogenboom, H. R., and Hudson, P. J. (2001) Design and application of diabodies, triabodies and tetrabodies for cancer targeting, Journal of Immunological Methods, 248, 47 - 66) is not observed for the aptamer dimer compounds in the biodistribution study. It can be ascribed to the smaller difference in molecular weight, a prolongation of the blood retention time should therefore be only a few minutes.

### Dynamic imaging studies with dimeric aptamer conjugates

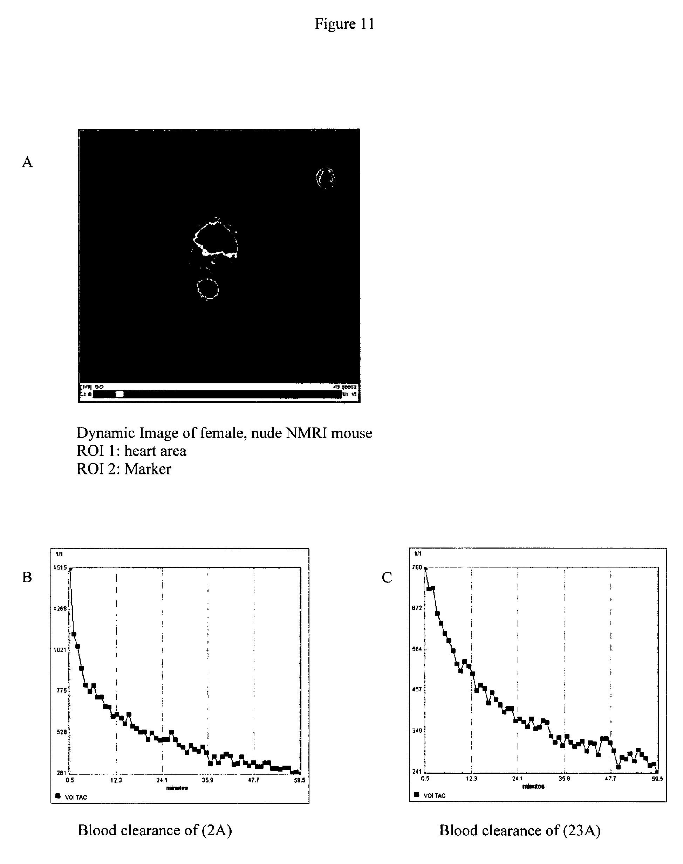

A doubling of the molecular weight should have an impact on the blood retention time and in order to revise if that holds true for the dimerized aptamer conjugate, the radiolabeled compounds (2A) and (23A) were compared in dynamic imaging study. Therefore, the radioactive probe was injected into the tail vein of the mice and subsequently the mice were imaged by planar scintigraphy. A region of interest (ROI) was placed over the area of the mouse's heart, which was put on a level with overall blood activity and the counts were recorded in frames of one minute. All together, 60 frames were measured and subsequently the counts of the single frames were transformed into a graph. The graphs of the two compounds illustrate the activity in the blood of the compounds (2A) and (23A) as a function of time and are depicted in Figure 11.

These graphs demonstrate a longer blood retention time for the radio labeled aptamer dimer. The half-life of the lead structure in the blood is about ten minutes while the retention of the dimer compound in the blood has a half-life of about twenty minutes. In this respect, the concept to prolong the blood retention time and to increase the tumor uptake was successful, it was proven that the molecular weight of a bio molecule is associated with its retention in the blood stream. However, the prolongation of the blood retention time is rather short and therefore the higher tumor uptake cannot be explained by this effect but rather by the avidity effect.

Particularly preferred embodiments
The present inventors have identified preferred structures for the preferred aptamer dimers, listed in Table 7 below.
"Pₙ" with n from 1-7 is a "peptidic linker" as defined previously.
All of the compounds of this invention can be prepared by methods described within the Examples, see above.

**Table 7**

| Dimer |
|---|
| (TTA1-MAG₂)₂P1 |
| (TTA1-MAG₂)₂P2 |
| (TTA1-MAG₂)₂P3 |
| (TTA1-MAG₂)₂P4 |
| (TTA1-MAG₂)₂P5 |
| (TTA1-MAG₂)₂P6 |
| (TTA1-MAG₂)₂P7 |
| (TTA3-MAG₂)₂P5 |

## Claims

1. A compound of the general formula (I)
Mal-L₁-(A)ₙ-C-(A)ₘ-L₂-Mal (formula I)
wherein
Mal is maleimide;
L₁ and L₂ each are absent or a linker structure selected from branched or unbranched, substituted or unsubstituted (C₁-C₁₂)Alkyl, (C₁-C₁₀Alkoxy, e.g. ethylene glycolₒ
wherein o is and integer from 1 to 12, wherein L₁ and L₂ can be the same or different; A is an amino acid or an derivative thereof;
n, m independently are selected from an integer between 0 and 20;
C is absent or a metal ion chelating group, or is a linking group for conjugating of a halogen bearing synthon;
wherein A and/or C independently can comprise one or more, identical or different protecting groups, and
pharmaceutically acceptable salts thereof.

2. The compound according to claim 1, wherein at least one of A is a D-amino acid.

3. The compound according to any of claims 1 or 2, wherein n is the same as m and is selected from 0, 1, 2, 3, 4, and 5.

4. The compound according to any of claims 1 to 3, wherein C is an N₃S chelator.

5. The compound according to any of claims 1 to 4, wherein L₁ is identical to L₂ and is-CH₂-CH₂-CO-.

6. The compound according to any claims 1 to 5 selected from
Mal-CH₂-CH₂-CO-βAla-Ser-Dpr-Met-Cys(tButhio)-Ser-Lys-CO-CH₂-CH₂-Mal;
Mal-CH₂-CH₂-CO-βAla-Ser-Dpr-Gly-Cys(tButhio)-Ser-Lys-CO-CH₂-CH₂-Mal;
Mal-CH₂-CH₂-CO-βAla-Ser-Ser-Dpr-Met-Cys(tButhio)-Ser-Ser-Lys-CO-CH₂-CH₂-Mal;
Mal-CH₂-CH₂-CO-βAta-Ser-Ser-Dpr-Gly-Cys(tButhio)-Ser-Ser-Lys-CO-CH₂-CH₂-Mal;
Mal-CH₂-CH₂-CO-βAla-Ser-Ser-Ser-Dpr-Met-Cys(tButhio)-Ser-Ser-Ser-Lys-CO-CH₂-CH₂-Mal;
Mal-CH₂-CH₂-CO-βAla-D-Ser-D-Ser-D-Ser-Dpr-Met-Cys(tButhio)-D-Ser-D-Ser-D-Ser-Lys-CO-CH₂-CH₂-Mal;
Mal-CH₂-CH₂-CO-βAla-D-Ser-D-Ser-D-Ser-Dpr-Gly-Cys(tButhio)-D-Ser-D-Ser-D-Ser-Lys-CO-CH₂-CH₂-Mal;
Mal-CH₂-CH₂-CO-βAla-Ser-Dpr-Met-Cys-Ser-Lys-CO-CH₂-CH₂-Mal;
Mal-CH₂-CH₂-CO-βAla-Ser-Dpr-Gly-Cys-Ser-Lys-CO-CH₂-CH₂-Mal;
Mal-CH₂-CH₂-CO-βAla-Ser-Ser-Dpr-Met-Cys-Ser-Ser-Lys-CO-CH₂-CH₂-Mal;
Mal-CH₂-CH₂-CO-βAla-Ser-Ser-Dpr-Gly-Cys-Ser-Ser-Lys-CO-CH₂-CH₂-Mal;
Mal-CH₂-CH₂-CO-βAla-Ser-Ser-Ser-Dpr-Met-Cys-Ser-Ser-Ser-Lys-CO-CH₂-CH₂-Mal;
Mal-CH₂-CH₂-CO-βAla-D-Ser-D-Ser-D-Ser-Dpr-Met-Cys-D-Ser-D-Ser-D-Ser-Lys-CO-CH₂-CH₂-Mal;
Mal-CH₂-CH₂-CO-βAla-D-Ser-D-Ser-D-Ser-Dpr-Gly-Cys-D-Ser-D-Ser-D-Ser-Lys-CO-CH₂-CH₂-Mal.

7. The compound according to any claims 1 to 6, wherein said compound is

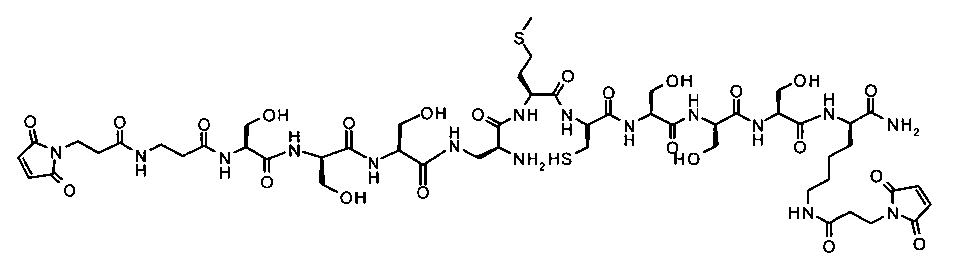

8. A compound of the general formula (II)
T₁-Mal-L₁-(A)ₙ-C-(A)ₘ-L₂-Mal-T₂ (formula II)
wherein
T₁ and T₂ each are a targeting group, wherein T₁ and T₂ independently can be identical or different;
Mal is maleimide;
L₁ and L₂ each are absent or a linker structure selected from branched or unbranched, substituted or unsubstituted (C₁-C₁₂)Alkyl, (C₁-C₁₂)Alkoxy, e.g. ethylene glycolₒ
wherein o is and integer from 1 to 12, wherein L₁ and L₂ can be the same or different;
A is an amino acid or an derivative thereof;
n, m independently are selected from an integer between 0 and 20;
C is absent or a metal ion chelating group, or is a linking group for conjugating of a halogen bearing synthon,
wherein A and/or C independently can comprise one or more, identical or different protecting groups, and
pharmaceutically acceptable salts thereof.

9. The compound according to claim 8, wherein at least one of T₁ and T₂ is an aptamer oligonucleotide.

10. The compound according to any of claims 8 or 9, wherein at least one of T₁ and T₂ is an aptamer oligonucleotide conjugated to an N₃S chelator.

11. The compound according to any of claims 8 to 10, wherein at least one of T₁ and T₂ is an aptamer oligonucleotide selected from the group consisting of aptamer oligonucleotides that bind to tenascin-C.

12. The compound according to any of claims 8 to 11, wherein at least one of T₁ and T₂ is an aptamer oligonucleotide selected from TTA1 and TTA3.

13. The compound according to any of claims 8 to 12, wherein the compound is selected from

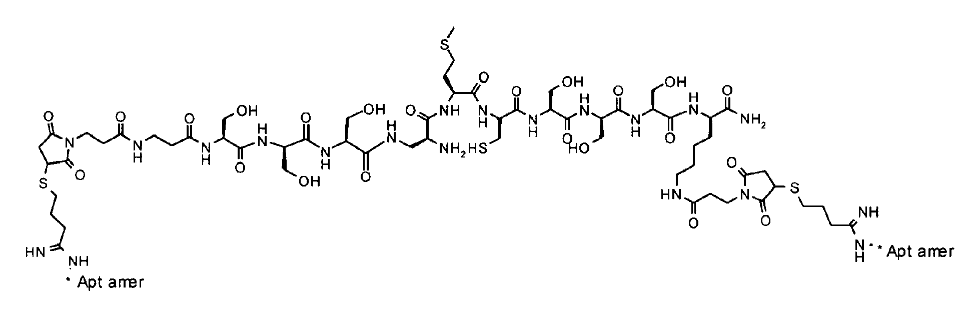

14. The compound according to any of claims 1 to 13, wherein the compound further comprises a metal ion.

15. The compound according to claim 14, wherein said metal ion is a radioactive isotope suitable for radio therapy or for diagnostic imaging.

16. The compound according to any of claims 14 to 15, wherein said metal ion is selected from the group consisting of ^{99m}Tc, ⁸⁶Y, ⁶⁸Ga, and ¹¹¹In.

17. A kit for preparing a radiopharmaceutical preparation, said kit comprising a vial comprising a quantity of the compound according to any of claims 1 to 13.

18. A method for producing a compound according to any of claims 1 to 7, comprising synthesis of said compound in an automated peptide synthesizer.

19. A method of producing a compound according to any of claims 8 to 13, comprising attaching said compound according to any of claims 1 to 7 through the maleimide group to a targeting moiety.

20. The compound according to any of claims 14 to 16 for use in therapy and/or diagnosis.

21. Use of the compound according to any of claims 1 to 16 for the manufacture of a medicament for the diagnosis and/or therapy of a proliferative disease.

22. A pharmaceutical or diagnostic composition, comprising a compound according to any of claims 1 to 16, and a pharmaceutically applicable carrier and/or excipient.
